# EUROPEAN PATENT APPLICATION

(11) **EP 0 771 878 A1**
(43) Date of publication of application: **07.05.1997**
(21) Application number: 95402425.3
(22) Date of filing: 31.10.1995
(51) Int. Cl.: C12N 15/82, C12N 15/54, A01H 5/00, A01H 5/10

(54) **Plants with reduced glucosinolate content**

(71) Applicant: PLANT GENETIC SYSTEMS N.V., 9000 Gent (BE); NATIONAL RESEARCH COUNCIL CANADA, Ottawa Ontario K1A OR6 (CA)
(72) Inventor: Van Audenhove, Katrien, B-8310 Assebroek (Brugge) (BE); Peferoen, Marnix, B-9850 Nevele (BE); Grootwassink, Jan Willem Derk, Saskatoon-Saskatchevan S7H 3A6 (CA); Underhill, Edward Wesley, Victoria-British Columbia V8V 2T4 (CA); Hemmingsen, Sean Mathias, Saskatoon-Saskatchewan S7H 3A2 (CA); Reed, Darwin Wilfred, Saskatoon-Saskatchewan S7M 4J9 (CA); Kolenovsky, Allan Dale, Saskatoon-Saskatchewan S7K 5C4 (CA)
(74) Representative: Desaix, Anne

(57) **Abstract**

The present invention relates to a method to reduce glucosinolate production and/or accumulation in plants wherein a reduced glucosinolate content is desired, particularly in the seeds of these plants. In accordance with the present invention, chimeric gene constructs inhibiting an enzyme responsible for glucosinolate production, the enzyme UDP-glucose:thiohydroximate S-glucosyltransferase (further referred to as "S-GT") are provided. Further in accordance with the present invention, plants are provided having substantially lower glucosinolate levels in their tissues, particularly in their seeds, which permit a significant expansion of the germplasm basis for the breeding of new oilseed rape varieties having lower glucosinolate levels, particularly in their seeds.

SEQUENCE LISTING

(1) GENERAL INFORMATION:

(i) APPLICANT:
(A) NAME: PLANT GENETIC SYSTEMS N.V.
(B) STREET: Plateaustraat 22
(C) CITY: Gent
(E) COUNTRY: BELGIUM
(F) POSTAL CODE (ZIP): B-9000
(G) TELEPHONE: 32 9 2358454
(H) TELEFAX: 32 9 2240694

(A) NAME: NATIONAL RESEARCH COUNCIL OF CANADA
(B) STREET: Montreal Road
(C) CITY: Ottowa
(D) STATE: Ontaria
(E) COUNTRY: CANADA
(F) POSTAL CODE (ZIP): K1A 0R6
(G) TELEPHONE: 1 613 9933899
(H) TELEFAX: 1 613 9526082

(ii) TITLE OF INVENTION: Plants with reduced glucosinolate content

(iii) NUMBER OF SEQUENCES: 29

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 31 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 1:

Val Thr Ile Ala Thr Thr Thr Tyr Thr Ala Ser Ser Ile Ser Thr Pro
1               5                   10                  15

Ser Val Ser Val Glu Pro Ile Ser Asp Gly His Asp Phe Ile Pro
            20                  25                  30

(2) INFORMATION FOR SEQ ID NO: 2:

(i) SEQUENCE CHARACTERISTICS:
(A) LENGTH: 14 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 2:

Ala Leu Gln Gln Ser Asn Phe Asn Phe Leu Trp Val Ile Lys
1               5                   10

(2) INFORMATION FOR SEQ ID NO: 3:

(i) SEQUENCE CHARACTERISTICS:
(A) LENGTH: 22 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 3:

Gly His Val Val Val Leu Pro Tyr Pro Val Gln Gly His Leu Asn Pro
1               5                   10                  15

Met Val Gln Phe Ala Lys
            20

(2) INFORMATION FOR SEQ ID NO: 4:

(i) SEQUENCE CHARACTERISTICS:
(A) LENGTH: 15 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 4:

Ala Thr Leu Ile Gly Pro Met Ile Asp Ser Ala Tyr Leu Asp Lys
1               5                   10                  15

(2) INFORMATION FOR SEQ ID NO: 5:

(i) SEQUENCE CHARACTERISTICS:
(A) LENGTH: 10 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 5:

Leu Pro Glu Gly Phe Val Glu Ala Thr Lys
1               5                   10

(2) INFORMATION FOR SEQ ID NO: 6:

(i) SEQUENCE CHARACTERISTICS:
(A) LENGTH: 7 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 6:

Phe Val Glu Glu Val Trp Lys
1               5

(2) INFORMATION FOR SEQ ID NO: 7:

(i) SEQUENCE CHARACTERISTICS:
(A) LENGTH: 21 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 7:

Ala Met Ser Glu Gly Gly Ser Ser Asp Arg Ser Ile Asn Glu Phe Val
1               5                   10                  15

Glu Ser Leu Gly Lys
            20

(2) INFORMATION FOR SEQ ID NO: 8:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 8:

CARGARWSNA AYTTYAAYTT                                                20

(2) INFORMATION FOR SEQ ID NO: 9:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 9:

CCNTAYCCNG TNCARGGNCA                                                20

(2) INFORMATION FOR SEQ ID NO: 10:

(i) SEQUENCE CHARACTERISTICS:
(A) LENGTH: 51 base pairs
(B) TYPE: nucleic acid
(C) STRANDEDNESS: single
(D) TOPOLOGY: linear

(xi) SEQUENCE DESCRIPTION: SEQ ID NO: 10:

CCTCTGAAGG TTCCAGAATC GATAGGAATT CTTTTTTTTT TTTTTTTTTV N 51

(2) INFORMATION FOR SEQ ID NO: 11:

(i) SEQUENCE CHARACTERISTICS:
(A) LENGTH: 38 base pairs
(B) TYPE: nucleic acid
(C) STRANDEDNESS: single
(D) TOPOLOGY: linear

(xi) SEQUENCE DESCRIPTION: SEQ ID NO: 11:

CTGGTTCGGC CCACCTCTGA AGGTTCCAGA ATCGATAG 38

(2) INFORMATION FOR SEQ ID NO: 12:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 12:

AAYTTYYTNT GGGTNATHAA 20

(2) INFORMATION FOR SEQ ID NO: 13:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 13:

CCNATGGTNC ARTTYGCNAA 20

(2) INFORMATION FOR SEQ ID NO: 14:

(i) SEQUENCE CHARACTERISTICS:
(A) LENGTH: 566 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(xi) SEQUENCE DESCRIPTION: SEQ ID NO: 14:

AGAAGCTCAT ATAGCGAAGT TACCAGAAGG GTTTGTGGAA GCTACCAAAG ACAGAGCGTT 60

GCTTGTTTCT TGGTGTAACC AGCTTGAGGT TTTAGCTCAT GGATCTATAG GTTGTTTTTT 120

GACTCACTGC GGTTGGAACT CGACGCTGGA AGGGTTGAGT TTGGGAGTTC CGATGGTGGG 180

TGTGCCGCAG TGGAGTGATC AGATGAATGA TGCTAAGTTT GTGGAGGAGG TTTGGAGAGT 240

TGGGTATAGA GCGAAAGATG AAGCTGGGGG AGGAGTTGTG AAGAGCGATG AGGTGGTGAG 300

GTGTTTGAAA GGAGTGATGG AAGGAGAGAG TAGTGTGGAG ATTAGAGAAA GTTCTAAGAA 360

ATGGAAAGAT TTGGCTGTGA AGGCGATGAG TGAAGGAGGA AGCTCTGATC GGAGCATTAA 420

TGAGTTTGTT GAGAGTTTAG GGAAGAAACA TTGAGAGGTA ATGAGATTTG TAAATCTTGT 480

GTGTTTGTTG TTGTTGCTCA AGAGCATTGT ACGGAGCGGA TTGTCATTCA GTAATATGAA 540

TAAACCAATT GTGATAGTAA AAAAAA 566

(2) INFORMATION FOR SEQ ID NO: 15:

(i) SEQUENCE CHARACTERISTICS:
(A) LENGTH: 568 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(xi) SEQUENCE DESCRIPTION: SEQ ID NO: 15:

AGAAGCTCAT ATAGCGAAGT TACCAGAAGG GTTTGTGGAA GCTACCAAAG ACAGAGCGTT 60

GCTTGTTTCT TGGTGTAACC AGCTTGAGGT TTTAGCTCAT GAATCGATAG GTTGCTTTTT 120

GACTCACTGC GGTTGGAACT CGACGTTGGA AGGATTGAGT TTGGGAGTTC CGATGGTTGG 180

TGTGCCTCAG TGGAGTGATC AGATGAATGA TGCTAAGTTT GTGGAGGAGG TTTGGAGAGT 240

TGGGTATAGA GCGAAGGAGG AAGCTGGGGG AGGAGTTGTG AAGAGCGATG AGGTGGTGAG 300

GTGTTTGAGA GGAGTGATGG AAGGAGAGAG TAGTGTGGAG ATTAGAGAGA GTTCTAAGAA 360

GTGGAAAGAT TTGGCTGTGA AGGCGATGAG TGAAGGAGGA AGCTCTGATC GGAGCATTAA 420

TGAGTTTGTG GAGAGTCTAG GGAAGAAACA TTGAGAGGTA ATGAGATTTG TAAATCTTGT 480

GTGTTTGTTG TTGTTGCTCA AGAGCATTGT ACGGAGCGGA TTGTCATTCA GTAATATGAG 540

TAAACCAATT GTGATATTTG AAAAAAAA 568

(2) INFORMATION FOR SEQ ID NO: 16:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 16:

GAYGGNCAYG AYTTYATHCC 20

(2) INFORMATION FOR SEQ ID NO: 17:

(i) SEQUENCE CHARACTERISTICS:
(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO: 17:

CCTTCCAGCG TCGAGTTCCA ACCGC 25

(2) INFORMATION FOR SEQ ID NO: 18:

(i) SEQUENCE CHARACTERISTICS:
(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO: 18:

CGGAATTCGC TAAAACCTCA AGCTGGTTAC ACC 33

(2) INFORMATION FOR SEQ ID NO: 19:

(i) SEQUENCE CHARACTERISTICS:
(A) LENGTH: 940 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(xi) SEQUENCE DESCRIPTION: SEQ ID NO: 19:

CACCTCAACC CAATGGTCCA GTTCGCTAAA CGCCTAGTCT CCAAAGGCCT CAAAGTCACA 60

ATCGCCACCA CCACCTACAC CGCCTCCTCC ATCTCCACCC CCTCCGTCTC CGTCGAACCA 120

ATCTCCGACG GCCACGACTT CATCCCCATA GGCGTCCCCG GCGTCAGCAT CGACGCCTAC 180

TCCGAATCCT TCAAGCTCCA CGGCTCCCAA ACCTTAACCC GCGTAATCTC CAAATTCAAA 240

TCCACAGATT CCCCCATCGA TTCTTTAGTC TACGACTCTT TCCTCCCGTG GGGACTCGAA 300

GTCGCGAGAT CCAACTCCCT CTCAGCTGCC GCTTTCTTCA CCAACAACCT CACCGTTTGC 360

TCTGTCCTTC GCAAATTCGC CTCCGGTGAG TTTCCTCTCC CCGCTGATCC CGCTTCCGCG 420

CTGTATCTCG TCCGTGGCTT GCCGGCTTTG AGCTACGACG AGCTTCCTTC CTTCGTGGGC 480

CGTCACTCGT CGAGCCACGC CGAACACGGG AGAGTTCTTC TGAACCAGTT CATTAACCAT 540

GAAGATGCTG ATTGGCTGTT CGTCAACGGC TTCGAAGGGT TAGAGACACA AGGTTGTGAA 600

GTTGGAGAAT CAGAGACTAT GAAGGCGACG TTGATCGGAC CTATGATCCC ATCTGCTTAT 660

CTTGACGCCC GAATCAAAGA CGATAAAGGC TACGGCTCGA GTCTGATGAA GCCGCTCTCG 720

GAGGAGTGTA TGGAGTGGTT AGACACTAAG CTGAGTAAGT CGGTGGTTTT TGTTTCGTTT 780

GGTTCCTTTA GGATCCTCTT TGAGAAGCAA CTAGCTGAGG TAGCAACGGC GTTACAAGAA 840

TCCAACTTTA ACTTCTTGTG GGTGATTAAA GAAGCTCGTA TAGCGAAGTT ACCAGAAGGG 900

TTTGTGGAAG CTACCAAAGA CAGAGCGTTG CTTGTTTCTT 940

(2) INFORMATION FOR SEQ ID NO: 20:

(i) SEQUENCE CHARACTERISTICS:
(A) LENGTH: 794 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(xi) SEQUENCE DESCRIPTION: SEQ ID NO: 20:

CATAGGCGTT CCCGGCGTCA GCATCGACGC ATACTCCGGA TCCTTCAAGC TCAACGGCTC 60

CGAAACCTTA ACCCGAGTAA TCTCAAAATT CAAATCCACA GATTCACCCA TCGATTCATT 120

AGTCTACGAC TCTTTCCTCC CGTGGGGACT CGAAGTCGCG AGATCTAACT CCATCTCAGC 180

TGCTGCTTTC TTCACCAACA ACCTCACCGT TTGCTCTGTT CTACGCAAAT TCGTCTCCGG 240

TGAGTTTCCT CTCCCCGCTG ATCCCGCTTC CGCGCCGTAT CTCGTCCGTG GCTTACCGGC 300

TTTGAGCTAC GACGAGCTTC CTTCCTTCGT CGGACGTCAC TCGTCGAGCC ACGCGGAGCA 360

CGGGAGAGTT CTTCTGAACC AGTTCCGTAA CCACGAAGAT GCTGATTGGC TGTTCGTCAA 420

CGGCTTCGAA GGGTTAGAGA CACAAGGTTG TGAAGTTGGA GAATCAGAGG CGATGAAGGC 480

GACGTTGATC GGACCTATGA TACCATCTGC TTATCTCGAC GGCCGAATCA AAGACGATAA 540

AGGCTACGGC TCGAGCCTGA TGAAGCCGCT CTCGGAGGAG TGTATGGAGT GGTTAGACAC 600

TAAGCTGAGC AAGTCGGTGG TTTTTGTTTC GTTTGGTTCC TTTGGGATCC TCTTTGAGAA 660

GCAACTCGCT GAGGTGGCAA AGGCGTTACA AGAATCCAAC TTTAACTTCT TGTGGGTGAT 720

CAAAGAAGCT CATATAGCGA AGTTACCAGA AGGGTTTGTG GAAGCTACCA AAGACAGAGC 780

GTTGCTTGTT TCTT 794

(2) INFORMATION FOR SEQ ID NO: 21:

(i) SEQUENCE CHARACTERISTICS:
(A) LENGTH: 26 base pairs
(B) TYPE: nucleic acid
(C) STRANDEDNESS: single
(D) TOPOLOGY: linear

(xi) SEQUENCE DESCRIPTION: SEQ ID NO: 21:

AGACGGAGGG GGTGGAGATG GAGGAG 26

(2) INFORMATION FOR SEQ ID NO: 22:

(i) SEQUENCE CHARACTERISTICS:
(A) LENGTH: 35 base pairs
(B) TYPE: nucleic acid
(C) STRANDEDNESS: single
(D) TOPOLOGY: linear

(xi) SEQUENCE DESCRIPTION: SEQ ID NO: 22:

CGGAATTCGG TGTAGGTGGT GGTGGCGATT GTGAC 35

(2) INFORMATION FOR SEQ ID NO: 23:

(i) SEQUENCE CHARACTERISTICS:
(A) LENGTH: 35 base pairs
(B) TYPE: nucleic acid
(C) STRANDEDNESS: single
(D) TOPOLOGY: linear

(xi) SEQUENCE DESCRIPTION: SEQ ID NO: 23:

CACGAATTCA CTATCGATTC TGGAACCTTC AGAGG 35

(2) INFORMATION FOR SEQ ID NO: 24:

(i) SEQUENCE CHARACTERISTICS:
(A) LENGTH: 38 base pairs
(B) TYPE: nucleic acid
(C) STRANDEDNESS: single
(D) TOPOLOGY: linear

(xi) SEQUENCE DESCRIPTION: SEQ ID NO: 24:

CTGGTTCGGC CCACCTCTGA AGGTTCCAGA ATCGATAG 38

(2) INFORMATION FOR SEQ ID NO: 25:

(i) SEQUENCE CHARACTERISTICS:
(A) LENGTH: 223 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(xi) SEQUENCE DESCRIPTION: SEQ ID NO: 25:

GATCAGACTT ATTATTTTTC TTCTTCCTCC TCCTCTTCTC AGTCTTCTTC AACTGAAAAC 60

AAACAGAAAC TAAGGCTTCA AAGTCACAAT GGTGGAAACA ACAACAACAA CAACAGCAAA 120

GACCAGCTCC AAAGGCCACG TCTTGGTCTT ACCTTACCCA GTCCAAGGCC ACCTCAACCC 180

AATGGTCCAG TTCGCTAAAC GCCTAGTCTC CAAAGGCCTC AAA 223

(2) INFORMATION FOR SEQ ID NO: 26:

(i) SEQUENCE CHARACTERISTICS:
(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO: 26:

TTATTTTTCT TCTTCCTCCT CCTCT 25

(2) INFORMATION FOR SEQ ID NO: 27:

(i) SEQUENCE CHARACTERISTICS:
(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO: 27:

AGCAACAACA ACAAACACAC AAGAT 25

(2) INFORMATION FOR SEQ ID NO: 28:

(i) SEQUENCE CHARACTERISTICS:
(A) LENGTH: 1513 base pairs
(B) TYPE: nucleic acid
(C) STRANDEDNESS: double
(D) TOPOLOGY: linear

(ii) MOLECULE TYPE: cDNA to mRNA

(ix) FEATURE:
(A) NAME/KEY: CDS
(B) LOCATION:69..1466

(xi) SEQUENCE DESCRIPTION: SEQ ID NO: 28:

TTATTTTTCT TCTTCCTCCT CCTCTGTGTC TTTGTCAACT GCAAACAAAA CAGAAACCAA 60

GTTCTGCA ATG GCG GAA ACA ACA ACA ACA ACA ACA GCG ACC AAC TCC AAA 110
Met Ala Glu Thr Thr Thr Thr Thr Thr Ala Thr Asn Ser Lys
1               5                   10

GGC CAC GTC GTG GTC TTA CCT TAC CCA GTC CAA GGC CAC CTC AAC CCA 158
Gly His Val Val Val Leu Pro Tyr Pro Val Gln Gly His Leu Asn Pro
15                  20                  25                  30

ATG GTC CAG TTC GCT AAA CGC CTA GTC TCC AAA GGC GTC AAA GTC ACA 206
Met Val Gln Phe Ala Lys Arg Leu Val Ser Lys Gly Val Lys Val Thr
                35                  40                  45

ATC GCC ACC ACC ACC TAC ACC GCC TCC TCC ATC TCC ACT CCC TCC GTC 254
Ile Ala Thr Thr Thr Tyr Thr Ala Ser Ser Ile Ser Thr Pro Ser Val
            50                  55                  60

TCC GTC GAA CCA ATC TCC GAC GGC CAC GAC TTC ATC CTC ATA GGC GTC 302
Ser Val Glu Pro Ile Ser Asp Gly His Asp Phe Ile Leu Ile Gly Val
        65                  70                  75

CCC GGC GTC AGC ATC GAC GCA TAC TCC GAA TCC TTC AAG CTC AAC GGC 350
Pro Gly Val Ser Ile Asp Ala Tyr Ser Glu Ser Phe Lys Leu Asn Gly
    80                  85                  90

TCC GAA ACC TTA ACC CGA GTA ATC TCA AAA TTC AAA TCC ACA GAT TCA 398
Ser Glu Thr Leu Thr Arg Val Ile Ser Lys Phe Lys Ser Thr Asp Ser
95                  100                 105                 110

CCC ATC GAT TCA TTA GTC TAC GAC TCT TTC CTC CCG TGG GGA CTC GAA 446
Pro Ile Asp Ser Leu Val Tyr Asp Ser Phe Leu Pro Trp Gly Leu Glu
                115                 120                 125

GTC GCG AGA TCT AAC TCC ATC TCA GCT GCT GCT TTC TTC ACC AAC AAC 494
Val Ala Arg Ser Asn Ser Ile Ser Ala Ala Ala Phe Phe Thr Asn Asn
            130                 135                 140

CTC ACC GTT TGC TCT GTT CTA CGC AAA TTC GTC TCC GGT GAG TTT CCT 542
Leu Thr Val Cys Ser Val Leu Arg Lys Phe Val Ser Gly Glu Phe Pro
        145                 150                 155

CTC CCC GCT GAT CCC GCT TCC GCG CCG TAT CTC GTC CGT GGC TTA CCG 590
Leu Pro Ala Asp Pro Ala Ser Ala Pro Tyr Leu Val Arg Gly Leu Pro
    160                 165                 170

GCT TTG AGC TAC GAC GAG CTT CCT TCC TTC GTC GGA CGT CAC TCG TCG 638
Ala Leu Ser Tyr Asp Glu Leu Pro Ser Phe Val Gly Arg His Ser Ser
175                 180                 185                 190

AGC CAC GCG GAA CAC GGG AGA GTT CTT CTG AAC CAG TTC CGT AAC CAC 686
Ser His Ala Glu His Gly Arg Val Leu Leu Asn Gln Phe Arg Asn His
                195                 200                 205

GAA GAT GCT GAT TGG CTG TTC GTC AAC AGC TTC GAA GGG TTA GAG ACA 734
Glu Asp Ala Asp Trp Leu Phe Val Asn Ser Phe Glu Gly Leu Glu Thr
            210                 215                 220

CAA GGT TGT GAA GTT GGA GAA TCA GAG GCG ATG AGG GCG ACG TTG ATC 782
Gln Gly Cys Glu Val Gly Glu Ser Glu Ala Met Arg Ala Thr Leu Ile
        225                 230                 235

GGA CCT ATG ATA CCA TCT GCT TAT CTC GAC GGC CGA ATC AAA GAC GAT 830
Gly Pro Met Ile Pro Ser Ala Tyr Leu Asp Gly Arg Ile Lys Asp Asp
    240                 245                 250

AAA GGC TAC GGC TCG AGC CTG ATG AAG CCG CTC TCG GAG GAG TGT ATG 878
Lys Gly Tyr Gly Ser Ser Leu Met Lys Pro Leu Ser Glu Glu Cys Met
255                 260                 265                 270

GAG TGG TTA GAC ACT AAG CTG AGC AAG TCG GTG GTT TTT GTT TCG TTT 926
Glu Trp Leu Asp Thr Lys Leu Ser Lys Ser Val Val Phe Val Ser Phe
                275                 280                 285

GGT TCC TTT GGG ATC CTC TTT GAG AAG CAA CTC GCT GAG GTG GCA AAG 974
Gly Ser Phe Gly Ile Leu Phe Glu Lys Gln Leu Ala Glu Val Ala Lys
            290                 295                 300

GCG TTA CAA GAA TCC AAC TTT AAC TTC TTG TGG GTG ATC AAA GAA GCT 1022
Ala Leu Gln Glu Ser Asn Phe Asn Phe Leu Trp Val Ile Lys Glu Ala
        305                 310                 315

CAT ATA GCG AAG TTA CCA GAA GGG TTT GTG GAA GCT ACC AAA GAC AGA 1070
His Ile Ala Lys Leu Pro Glu Gly Phe Val Glu Ala Thr Lys Asp Arg
    320                 325                 330

GCG TTG CTT GTT TCT TGG TGT AAC CAG CTT GAG GTT TTA GCT CAT GTA     1118
Ala Leu Leu Val Ser Trp Cys Asn Gln Leu Glu Val Leu Ala His Val
335                 340                 345                 350

TCG ATA GGT TGC TTT TTG ACT CAC TGC GGT TGG AAC TCG ACG TTG GAA     1166
Ser Ile Gly Cys Phe Leu Thr His Cys Gly Trp Asn Ser Thr Leu Glu
                355                 360                 365

GGA TTG AGT TTG GGA GTT CCG ATG GTT GGT GTG CCT CAG TGG AGT GAT     1214
Gly Leu Ser Leu Gly Val Pro Met Val Gly Val Pro Gln Trp Ser Asp
            370                 375                 380

CAG ATG AAT GAT GCT AAG TTT GTG GAG GAG GTT TGG AGA GTT GGG TAT     1262
Gln Met Asn Asp Ala Lys Phe Val Glu Glu Val Trp Arg Val Gly Tyr
        385                 390                 395

AGA GCG AAG GAG GAA GCT GGG GGA GGA GTT GTG AAG AGC GAT GAG GTG     1310
Arg Ala Lys Glu Glu Ala Gly Gly Gly Val Val Lys Ser Asp Glu Val
    400                 405                 410

GTG AGG TGT TTG AGA GGA GTG ATG GAA GGA GAG AGT AGT GTG GAG ATT     1358
Val Arg Cys Leu Arg Gly Val Met Glu Gly Glu Ser Ser Val Glu Ile
415                 420                 425                 430

AGA GAG AGT TCT AAG AAG TGG AAA GAT TTG GCT GTG AAG GCG ATG AGT     1406
Arg Glu Ser Ser Lys Lys Trp Lys Asp Leu Ala Val Lys Ala Met Ser
                435                 440                 445

GAA GGA GGA AGC TCT GAT CGG AGC ATT AAT GAG TTT GTG GAG AGT CTA     1454
Glu Gly Gly Ser Ser Asp Arg Ser Ile Asn Glu Phe Val Glu Ser Leu
            450                 455                 460

GGG AAG AAA CAT TGAGAGGTAA TGAGATTTGT AAATCTTGTG TGTTTGTTGT     1506
Gly Lys Lys His
        465

TGTTGCT                                                         1513

(2) INFORMATION FOR SEQ ID NO: 29:

    (i) SEQUENCE CHARACTERISTICS:
        (A) LENGTH: 466 amino acids
        (B) TYPE: amino acid
        (D) TOPOLOGY: linear

    (ii) MOLECULE TYPE: protein
    (xi) SEQUENCE DESCRIPTION: SEQ ID NO: 29:

Met Ala Glu Thr Thr Thr Thr Thr Thr Ala Thr Asn Ser Lys Gly His
  1               5                  10                  15

Val Val Val Leu Pro Tyr Pro Val Gln Gly His Leu Asn Pro Met Val
             20                  25                  30

Gln Phe Ala Lys Arg Leu Val Ser Lys Gly Val Lys Val Thr Ile Ala
         35                  40                  45

Thr Thr Thr Tyr Thr Ala Ser Ser Ile Ser Thr Pro Ser Val Ser Val
     50                  55                  60

Glu Pro Ile Ser Asp Gly His Asp Phe Ile Leu Ile Gly Val Pro Gly
 65                  70                  75                  80

Val Ser Ile Asp Ala Tyr Ser Glu Ser Phe Lys Leu Asn Gly Ser Glu
                 85                  90                  95

Thr Leu Thr Arg Val Ile Ser Lys Phe Lys Ser Thr Asp Ser Pro Ile
            100                 105                 110

Asp Ser Leu Val Tyr Asp Ser Phe Leu Pro Trp Gly Leu Glu Val Ala
        115                 120                 125

Arg Ser Asn Ser Ile Ser Ala Ala Ala Phe Phe Thr Asn Asn Leu Thr
    130                 135                 140

Val Cys Ser Val Leu Arg Lys Phe Val Ser Gly Glu Phe Pro Leu Pro
145                 150                 155                 160

Ala Asp Pro Ala Ser Ala Pro Tyr Leu Val Arg Gly Leu Pro Ala Leu
                165                 170                 175

Ser Tyr Asp Glu Leu Pro Ser Phe Val Gly Arg His Ser Ser Ser His
            180                 185                 190

Ala Glu His Gly Arg Val Leu Leu Asn Gln Phe Arg Asn His Glu Asp
        195                 200                 205

Ala Asp Trp Leu Phe Val Asn Ser Phe Glu Gly Leu Glu Thr Gln Gly
    210                 215                 220

Cys Glu Val Gly Glu Ser Glu Ala Met Arg Ala Thr Leu Ile Gly Pro
225                 230                 235                 240

Met Ile Pro Ser Ala Tyr Leu Asp Gly Arg Ile Lys Asp Asp Lys Gly
                245                 250                 255

Tyr Gly Ser Ser Leu Met Lys Pro Leu Ser Glu Glu Cys Met Glu Trp
            260                 265                 270

Leu Asp Thr Lys Leu Ser Lys Ser Val Val Phe Val Ser Phe Gly Ser
        275                 280                 285

Phe Gly Ile Leu Phe Glu Lys Gln Leu Ala Glu Val Ala Lys Ala Leu
    290                 295                 300

Gln Glu Ser Asn Phe Asn Phe Leu Trp Val Ile Lys Glu Ala His Ile
305                 310                 315                 320

Ala Lys Leu Pro Glu Gly Phe Val Glu Ala Thr Lys Asp Arg Ala Leu
                325                 330                 335

Leu Val Ser Trp Cys Asn Gln Leu Glu Val Leu Ala His Val Ser Ile
            340                 345                 350

Gly Cys Phe Leu Thr His Cys Gly Trp Asn Ser Thr Leu Glu Gly Leu
        355                 360                 365

Ser Leu Gly Val Pro Met Val Gly Val Pro Gln Trp Ser Asp Gln Met
    370                 375                 380

Asn Asp Ala Lys Phe Val Glu Glu Val Trp Arg Val Gly Tyr Arg Ala
385                 390                 395                 400

Lys Glu Glu Ala Gly Gly Gly Val Val Lys Ser Asp Glu Val Val Arg
                405                 410                 415

Cys Leu Arg Gly Val Met Glu Gly Glu Ser Ser Val Glu Ile Arg Glu
            420                 425                 430

Ser Ser Lys Lys Trp Lys Asp Leu Ala Val Lys Ala Met Ser Glu Gly
        435                 440                 445

Gly Ser Ser Asp Arg Ser Ile Asn Glu Phe Val Glu Ser Leu Gly Lys
    450                 455                 460

Lys His
465

## Description

### Field of the invention

The present invention relates to a method to reduce glucosinolate production and/or accumulation in plants wherein a reduced glucosinolate content is desired, particularly in the seeds of these plants. In accordance with the present invention, chimeric gene constructs inhibiting an enzyme responsible for glucosinolate production, the enzyme UDP-glucose:thiohydroximate S-glucosyltransferase (further referred to as "S-GT") are provided. Further in accordance with the present invention, plants are provided having substantially lower glucosinolate levels in their tissues, particularly in their seeds, which permit a significant expansion of the germplasm basis for the breeding of new oilseed rape varieties having lower glucosinolate levels, particularly in their seeds.

### Background

Glucosinolates are low molecular weight sulphur-containing glucosides that are produced and stored in almost all tissues of members of the Capparales, the most important member being the group of Crucifer plants (Haughn et al., 1991, Plant Physiol. 97, 217-226). They are composed of two parts, a glycone moiety and a variable aglycone side chain derived from α-amino acids. When intact, these secondary metabolites are totally passive and innocuous, and do not have any known physiological function. During the mechanical breaking of plant tissues, e.g., during consumption, the glucosinolates come into contact with an endogenous enzyme, myrosinase. The resulting breakdown products include isothiocyanates, nitriles, thiocyanates, isocyanates, thiones and alcohols. Intake of large amounts of glucosinolates and their breakdown products cause acute goiter and chronic disease in experimental and food-producing animals (Bell, 1993, Can. J. Anim. Sci. 73, 679-697).

From an agronomical point of view, the most important part of an oilseed rape crop are the seeds, which are used to extract the oil. The seed cake is a protein-rich animal feed, which remains after extraction of the oil from the seeds. The presence of glucosinolates in the seed cake is undesired in view of the known toxicity of glucosinolate-breakdown products on animals and humans. Certainly for oilseed crops such as mustard and oilseed rape, the presence of these glucosinolate-breakdown products is problematic. Therefore, breeding programs have been set up to decrease the amount of glucosinolates in these crops, particularly in oilseed rape, to an acceptable level. Unfortunately, however, glucosinolate production in *Brassica napus* appears to be a multigenic trait.

In Canada, the term "canola" describes oilseed rape with limited levels of glucosinolates and erucic acid in the harvested seeds. Conventional plant breeding efforts have in exceptional cases already resulted in some "zero" glucosinolate oilseed rape varieties.

The cDNA sequence of a B. napus myrosinase, an enzyme breaking down glucosinolates, has been described (Falk et al., 1992, Plant Science 83, 181-186). It has been suggested to target the myrosinase to the storage sites of the glucosinolates in the plant cells so as to prematurely destroy them (GrootWassink, 1994a, PBI Bulletin (National Research Council of Canada)).

Furthermore, several attempts have been made to isolate a pure S-GT enzyme from several Crucifers, but these were not successful, since the S-GT enzyme occurs at very low levels, most of the activity is lost upon purification, the enzyme is instable and the enzyme tends to associate with other materials released from the cells (Reed et al. (Arch. Biochem. Biophys. 305, 526-532, 1993); Guo et al. (Phytochemistry 36, 1133-1138, 1994)). Although GrootWassink et al. (1994b, Plant Physiol. 105, 425-433) describe the immunopurification of S-GT from the florets of Brassica olearaceae spp. botrytis (cauliflower), this method resulted in a protein containing several S-GT isoenzymes with different pI values. Thus, the purification to homogeneity of a sole S-GT enzyme has not been accomplished to date.

It has also been suggested to put the gene for the S-glucosyltransferase enzyme back into canola, but in reverse orientation so that the antisense DNA strand is transcribed (Poulton and Moller, 1993, In Methods in Plant Biochemistry, vol. 9. Academic Press, London, pp. 209-237; GrootWassink, 1994a, supra). However, this suggestion was never accomplished since the isolation or cloning of a DNA sequence encoding an S-GT enzyme has never been reported.

Chavadej et al. (1994, Prot. National Acad. Science USA 91, 2166-2170) expressed a tryptophan decarboxylase in transgenic oilseed rape plants to obtain a significant decrease in the levels of indole glucosinolates. However, the levels of other glucosinolates remained unaltered.

Accordingly, it is an object of the present invention to overcome the problems known in the art by providing a pure S-GT enzyme, the DNA sequence encoding it and a method to reduce glucosinolate production and/or accumulation in plants. These and other objects are achieved by the present invention as evidenced by the summary of the invention, description of the preferred embodiments and the claims.

### Summary of the invention

Accordingly, it is an object of the present invention to provide a method for reducing the S-GT activity in plant cells, by reducing the expression of the s-gt isoforms genes.

Yet another object of the present invention is to provide plant-expressible chimeric genes and transformation vectors comprising an s-gt inhibitory chimeric gene such as a gene encoding an antisense s-gt RNA. In a preferred embodiment, the present invention provides a plant expressible chimeric gene comprising a gene encoding an antisense RNA complementary to all or part of the RNA encoded by the plant s-gt gene, the cDNA of which is comprised in clone pGL9, which is deposited at the BCCM-LMBP under accession number 3344.

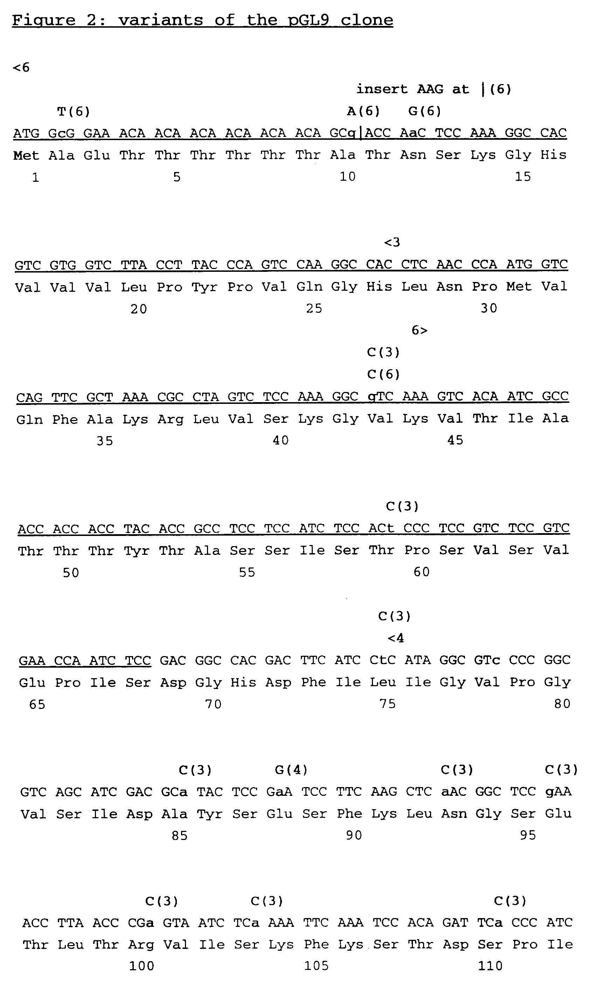

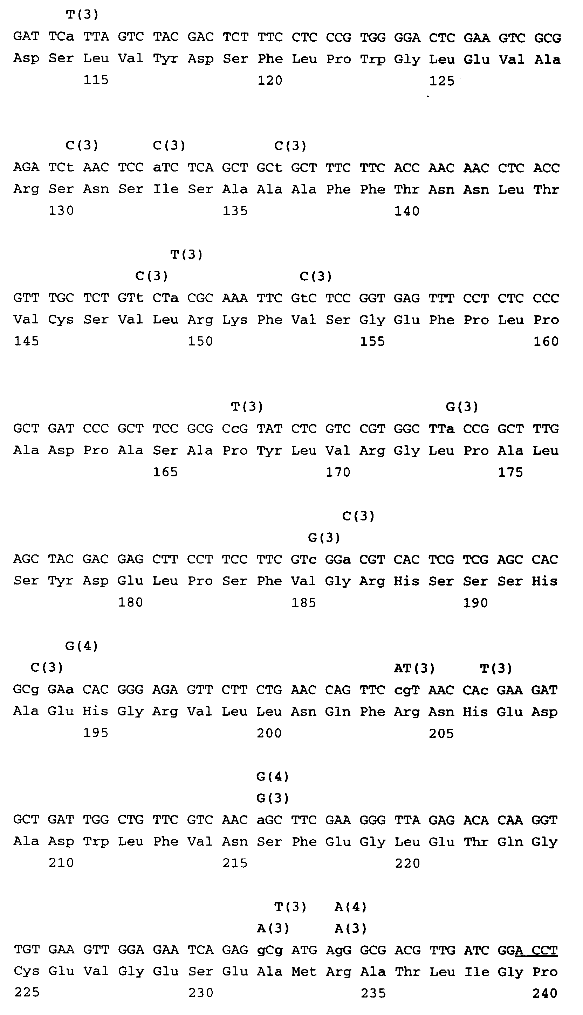

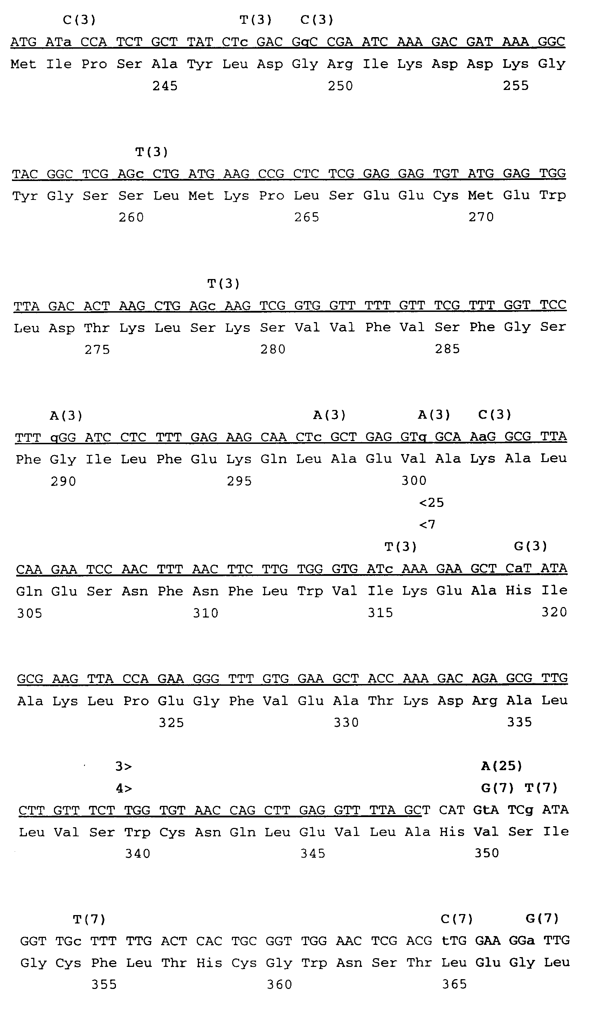

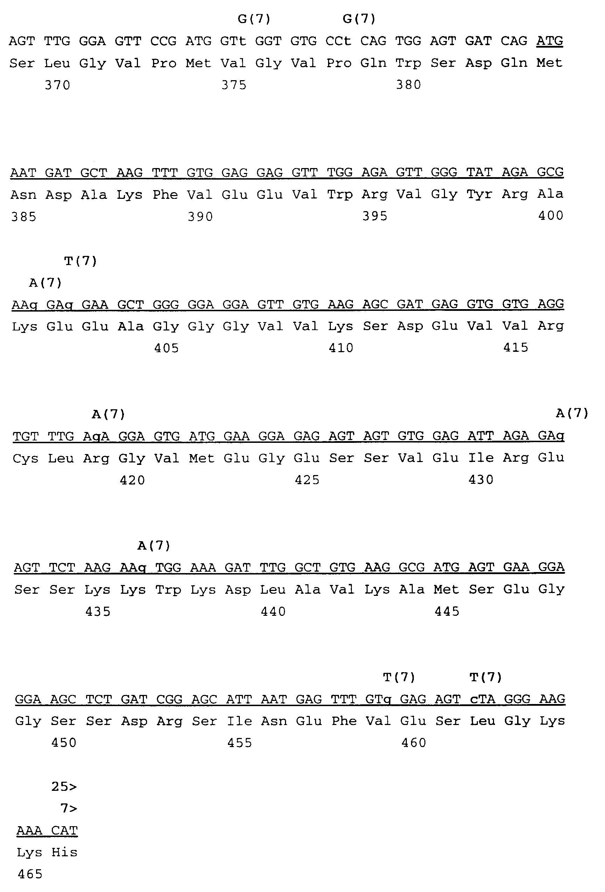

Yet another preferred embodiment of the present invention provides a chimeric gene encoding an antisense RNA complementary to all or part (at least 100 nucleotides) of the RNA, a cDNA of which comprises the DNA sequence of SEQ ID No. 28, or an antisense RNA complementary to the RNA encoded by a variant of the s-gt gene, coding for a protein with substantially the same S-GT activity, such as any of the DNA sequences represented in Figure 2.

In yet another aspect, the present invention provides a plant, transformed to contain a chimeric gene comprising :
(a) a plant-expressible promoter ;
(b) a transcribed region operably linked to said promoter, comprising a DNA sequence encoding an RNA or protein, wherein said RNA or protein interferes with the normal expression of the UDP-glucose:thiohydroximate S-glucosyltransferase gene (s-gt gene) in cells of said plant; and
(c) a 3' transcription termination and polyadenylation region active in said plant; as well as seeds, and seed cakes obtained from said seeds.

Yet another aspect of the present invention provides a DNA comprising a region encoding a protein with UDP-glucose:thiohydroximate S-glucosyltransferase activity, selected from the following:
1) a DNA encoding an mRNA, the cDNA of which is contained in plasmid pGL9, deposited in E. coli WK6 at the BCCM-LMBP under accession number 3344;
2) a DNA encoding an mRNA, the cDNA of which has the sequence of SEQ ID No. 28;
3) a DNA having substantial sequence homology to SEQ ID No:28.

Yet another aspect of the present invention provides a DNA sequence encoding an antisense RNA selected from the following:
1) an antisense RNA which is complementary, preferably at least 90 % complementary, more preferably at least 95 % complementary, to a region of at least 100 nucleotides, preferably a region of at least 500 nucleotides, of an mRNA, the cDNA of which is contained in plasmid pGL9, deposited in E. coli WK6 at the BCCM-LMBP under accession number 3344;
2) an antisense RNA, which is at least 90 % complementary, more preferably at least 95 % complementary, to a region of at least 100 nucleotides, preferably a region of at least 500 nucleotides, of an mRNA, the cDNA of which comprises the coding region of SEQ ID No. 28; or
3) an antisense RNA encoded by the s-gt inhibitory gene contained in plasmid TKV8a included in E. coli, MC1061, deposited a the BCCM-LMBP under accession number LMBP 3343, or an RNA having substantial sequence homology thereto.

Yet another aspect of the present invention provides a process for obtaining a Brassica napus plant having a significantly reduced expression of an s-gt gene, comprising the following steps:
a) transforming a plant cell with a s-gt inhibitory chimeric gene; and
b) regenerating a plant from said transformed cell.

Also contemplated by the present invention are hybrid plants having a glucosinolate content of less than 30 µmoles per gram dry defatted seed glucosinolates in their seeds.

### Brief Description of the Drawings

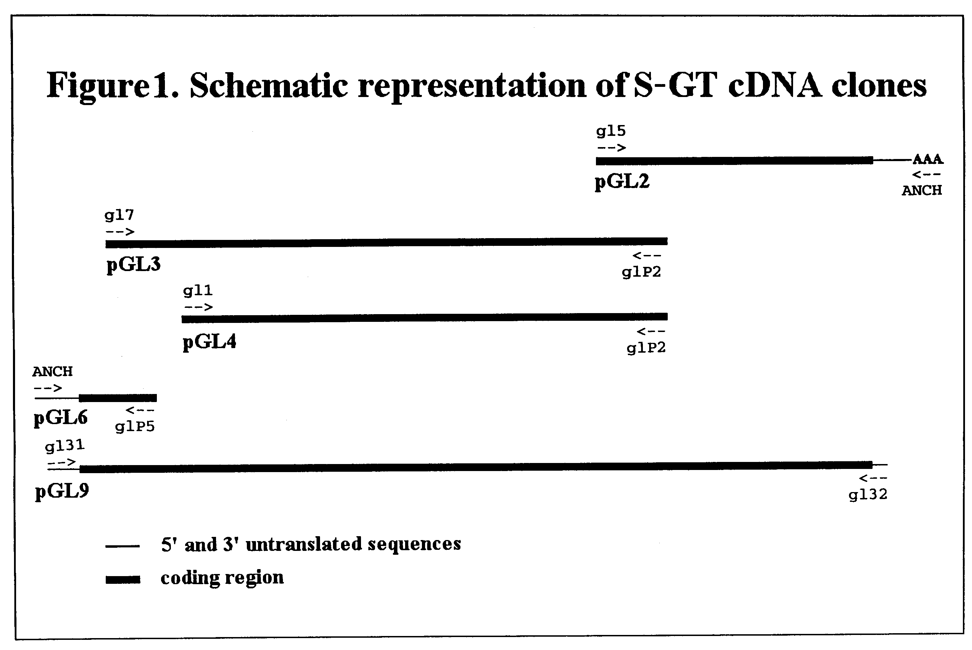

Fig.1 is a schematic representation of the different cDNA clones obtained from B. napus S-GT mRNA and their delineation on the full cDNA obtained. The thick lines represent the coding region, the thinner lines represent the 5' and 3' untranslated sequences of the cDNA. The primers used to obtain the cDNAs are indicated, with their corresponding direction ("ANCH" refers to the 5' or 3' Anchor primers (Clontech) described below, locations are approximate and the arrows representing the primers are not drawn to scale).

Fig. 2 is the ORF of the pGL9 clone of SEQ ID No. 28 indicating all nucleotide differences found in the coding regions of the different s-gt cDNAs isolated in accordance with this invention. The altered nucleotide in the coding regions of the other cDNAs is indicated above the DNA sequence of the pGL9 clone, the corresponding nucleotide in the DNA sequence of pGL9 is in lowercase letters. The numbers between brackets are as follows: (6) refers to the pGL6-14 clone, (3) to pGL3-22, (4) to pGL4-2, (7) to pGL2-7, (25) to pGL2-25. The underlined parts were found to be identical between pGL18 clone 1 and the pGL9 clone. No amino acid differences are indicated in the Figure. The 5' and 3' ends of the open reading frames contained in the different cDNA clones are also shown above the pGL9 sequence by the marks < and >, respectively (the consecutive numberings refer to amino acid positions).

### Detailed Description of the Preferred Embodiments of the Invention

The present invention provides a method to reduce glucosinolate production and/or accumulation in plants. This method was achieved by isolating DNA sequences encoding a Brassica napus S-GT enzyme form. Starting from the partial amino acid sequences of peptide fragments obtained from the purified B. oleraceae S-GT, degenerate DNA primers were designed and DNA fragments encoding all or part of an S-GT enzyme were isolated by PCR-RACE.

The following definitions are provided to further clarify the terminology used throughout the specification, it being understood that these definitions are provided for the person skilled in the art to solely use as a basis for interpreting the preferred embodiments, the examples and the claims.

As used herein, "substantial sequence similarity", refers to DNA sequences encoding similar RNAs and/or proteins with some differences in their RNA or amino acid sequence, e.g., nucleotide or amino acid deletions, additions, or replacements, with the proviso that these similar RNAs and/or proteins still retain significantly the same function or activity. Preferably, DNA sequences with "substantial sequence similarity" encode proteins having the same tertiary structure in those domains or regions determining the protein activity. Also encompassed in the definition of "substantial sequence similarity", when referring to DNA sequences, are different DNA sequences encoding the same proteins. Indeed, because of the degeneracy of the genetic code, many different DNA sequences can encode one protein. Also, during cloning work some nucleotides can be changed to create suitable restriction sites throughout a DNA sequence, or introns can be inserted, while retaining substantial sequence similarity. These nucleotide changes made during cloning are also encompassed by the term "substantial sequence similarity".

Furthermore, natural variants having substantial sequence similarity to a DNA sequence differing in some nucleotides or synthetic variants having substantial sequence similarity which can be made by recombinant DNA techniques are encompassed by the definition of "substantial sequence similarity".

In a preferred embodiment of this invention, DNA sequences have substantial sequence similarity if they have more than 85 %, preferably more than 90 %, more preferably more than 95 %, sequence similarity.

Sequence similarity between two nucleotide sequences is conveniently measured by the Wilbur and Lipmann algorithm with the IntelliGenetics™ (Intelligenetics Inc.) sequence analysis package (Wilbur and Lipmann, 1983, Proc. Natl. Acad. Sci. USA 80, 726) using a window-size of 20 nucleotides, a word length of 4 nucleotides and a gap penalty of 4.

It is known that some amino acids in a protein can be replaced by others, provided the tertiary structure is not significantly altered. Therefore, for proteins, "substantial sequence similarity" refers to proteins differing in some amino acids, e.g., by amino acid deletions, additions or replacements, while retaining the same overall function as determined by the proteins tertiary structure. Typically, amino acid differences between functionally active protein forms with substantial sequence similarity are below 5 %, preferably below 3 %, of the total number of amino acids. Sequence similarity between two protein sequences can be conveniently measured by the Wilbur and Lipmann algorithm with the IntelliGenetics™ (Intelligenetics Inc.) sequence analysis package (Wilbur and Lipman, 1983, supra) using a window size of 20 amino acids, a word length of 2 amino acids, and a gap penalty of 4.

A "chimeric gene", as used herein, is a gene wherein at least one regulatory region is heterologous to and therefore not normally associated with the coding region or the transcribed region in nature; e.g., an s-gt coding region under the control of a bacterial promoter.

As used herein, "a plant-expressible chimeric gene" is a gene expressible in cells of a plant, comprising at least one regulatory region; e.g., a plant-expressible promoter or a 3' transcription termination and polyadenylation sequence active in plant cells, which is not normally associated with the transcribed region or the coding region in plant cells in nature. A plant-expressible chimeric gene in accordance with this invention typically encodes an RNA which is translated into a protein, or an RNA which is functional as such, e.g., an antisense RNA or a ribozyme. A DNA encoding an (antisense) RNA, complementary to a (sense) RNA produced in a plant cell, operably linked to the promoter and 3' transcription termination and polyadenylation region normally regulating transcription of the (sense) RNA, is also included under the definition of chimeric gene in accordance with this invention.

As used herein, "plant-expressible promoter" is a promoter active in plant cells, including but not limited to promoters of plant origin and of bacterial or viral origin that are functional in plant cells (e.g., CaMV 35S promoter, Agrobacterium T-DNA promoters and the like).

As used herein, "promoter" is a nucleotide sequence recognized (directly or indirectly) and bound by DNA-dependent RNA polymerase during initiation of transcription.

As used herein, "promoter region" is a DNA sequence typically located 5' of a coding region, and including the promoter and a 5' untranslated leader sequence.

As used herein, "transcribed region" is that region of a DNA transcribed into an RNA, typically comprising 5' leader sequences, a coding region (which, by definition includes a region encoding a protein or a region encoding an antisense RNA or a ribozyme) and 3' untranslated trailer sequences.

As used herein, "antisense RNA" is an RNA molecule which, by binding to (hybridizing to) a complementary sequence in another nucleic acid molecule (RNA or DNA), inhibits the function and/or completion of synthesis of the complementary molecule. Antisense RNA is typically produced from a chimeric gene by operably linking all or part of a DNA sequence encoding an RNA sequence to a promoter in the orientation opposite to the orientation in which the DNA encoding the RNA (or its part) is operably linked to its promoter in an endogenous plant gene, so that an RNA is formed which is complementary to all or part of the RNA normally produced from the endogenous gene in a plant cell. Antisense RNA can comprise a sequence complementary to all or part of the 5' and 3' untranslated regions of an RNA to be inactivated, or even a sequence complementary to introns or parts of introns of a pre-mRNA, such that the antisense is rendered more specific. The antisense RNA is at least 85 %, preferably at least 90 %, most preferably 95 to 100 %, complementary to the RNA to be inhibited.

The term "complementary", as used herein, refers to a sequence of nucleotide bases in one strand of a DNA or RNA molecule that is exactly complementary to that on another single strand such that there is no variation between the adenine-thymidine, adenine-uracil or guanine-cytosine base pairs.

As used herein, "90 % complementary" refers to an RNA sequence wherein 90 % of the nucleotides are complementary to the corresponding nucleotides in another RNA sequence, preferably of the same length, so that only 10 % of the nucleotides will not hybridize to the corresponding nucleotide on the other RNA or DNA. For example, for two RNAs with the same number of nucleotides, when RNA sequence 1 is 90 % complementary to an RNA sequence 2, the complementary form of RNA sequence 1 will have 90 % sequence similarity with RNA sequence 2.

"S-GT", or "S-GT protein" as used herein, refers to a member of the UDP-glucose:thiohydroximate S-glucosyltransferase enzyme family (EC 2.4.1.-) of plants, particularly Brassica plants, that comprises the enzyme forms which catalyze the penultimate step in glucosinolate production.

"s-gt gene" or "s-gt DNA", as used herein, refers to a DNA sequence encoding an S-GT protein.

As used herein, "functionally equivalent parts" of a DNA, RNA or protein are portions of a DNA, RNA or protein which have the same function or activity as the full DNA, RNA or protein. For example, a functionally equivalent part of an antisense RNA, is a portion of an antisense RNA, wherein this portion exhibits substantially the same antisense (inhibitory) effect as the full length antisense RNA, although the portion will differ from the full length antisense RNA in certain other characteristics such as molecular weight, its size, its relative nucleotide composition and the like. Also, a protein fragment, which has the same metabolic activity (e.g., glucosyltransferase activity in plant cells) as the entire protein, is a "functionally equivalent part" of that protein in accordance with this invention.

As used herein, "gene silencing" is a significant or complete reduction in detectable gene expression. Gene silencing can be achieved at any level of gene expression, and results in a significant drop of production of RNA or protein. In a preferred embodiment, gene silencing is achieved by using a DNA encoding an antisense RNA or a functionally effective part thereof.

As used herein, "partial gene silencing" refers to an inhibition of expression of less than 100 %, preferably from about 50 to about 95 %, as measured by RNA or protein levels, while "complete gene silencing" refers to the situation wherein no detectable gene expression (RNA or protein) is observed.

As used herein, a "ribozyme" is a catalytic RNA molecule capable of specifically cleaving another RNA. A ribozyme typically has a "targetting" sequence which is complementary to another RNA, so that the ribozyme can recognize and cleave this other RNA. The targetting sequence preferably is at least 90 %, more particularly at least 95 %, preferably 100 %, complementary to the RNA which is to be cleaved.

As used herein, the terms "significantly reduced", "significant inhibition", or "significantly lower levels", when referring to s-gt gene expression, refer to a quantitative difference in expression of the native s-gt gene in a plant cell transformed with an s-gt inhibitory chimeric gene when compared to the situation in the wild-type plant, as is evidenced by protein levels measured via quantitative protein assays (e.g., ELISA), in a plant cell. Preferably, significantly reduced or inhibited expression of an s-gt gene in accordance with this invention refers to a reduction in formation of S-GT protein of 50% to 95%, particularly at least 75%, more particularly at least 85%, preferably 95%, in a plant cell as is measured by protein quantitative assays in comparison to a cell of the same cell type used as a control, or by s-glucosyl transferase activity as measured by GrootWassink et al, 1994b *supra*.

"Reduced total glucosinolate", as used herein, refers to a reduction in total glucosinolate levels to below 30 µmoles per gram oil free seed matter, preferably below 10, more preferably below 5 µmoles per gram oil free seed matter, particularly to undetectable levels using the method of GrootWassink et al (1994b).

In accordance with this invention, DNA sequences encoding S-GT proteins have been isolated. All or part of the isolated s-gt DNA, preferably the s-gt coding region, can be used in a variety of ways, including but not limited to the production of S-GT protein in bacteria, the use of the isolated s-gt DNA in chimeric genes, plants transformed by the chimeric genes according to the invention, the production of seeds lacking or having reduced content of glucosinolates and the production of seed cakes obtained by the crushing of the seeds.

To produce S-GT protein free from contaminating plant proteins, all or part of the isolated s-gt DNA and preferably the s-gt coding region can be used. Preferred promoter and 3' transcription termination sequences for the chimeric s-gt gene to be expressed in bacteria are derived from bacterial genes (see, Sambrook et al, Molecular Cloning, A Laboratory Manual (1989)). The s-gt cDNA which corresponds to the full open reading frame of an s-gt gene (and which is contained in plasmid pGL9 (BCCM-LMBP 3344)), is cloned into an E. coli expression vector, by methods known in the art, to recombinantly produce the protein. The activity of the recombinantly produced S-GT enzyme is confirmed by assaying for glucosyl-transferase-activity using the assay as described by Reed et al. (1993, supra) and GrootWassink et al. (1994b, supra).

A DNA sequence encoding a B. napus S-GT enzyme as shown in SEQ ID No 28. Nevertheless, other different isoforms of the gene exist, having some amino acids differences. Some amino acids were found to be different in the B. napus clone when compared to the peptide fragments obtained from the B. oleraceae S-GT form. It is clear from the Examples and from Figure 2, that also in B. napus, evidence for the existence of isozymes differing in some amino acid residues was found. This indicates that a small family of related s-gt genes having substantial sequence similarity exists in Brassica plants.

The following amino acids were found to be different between the S-GT protein of SEQ ID No. 28, the B. oleraceae protein and the other isoforms, of which a cDNA clone was isolated. The amino acid which is different at this position in an isoform, and, between brackets, the amino acid in SEQ ID No. 28 as indicated below; position 2: Val(Ala); between position 10-11: add Lys between amino acids 10 and 11; position 12: Ser(Asn); position 43: Leu(Val); position 75: Pro(Leu); position 88: Gly(Glu); position 93: His(Asn); position 96: Gln(Glu); position 133: Leu(Ile); position 153: Ala(Val); position 167: Leu(Pro); position 204: Ile(Arg); position 216: Gly(Ser); position 232: Thr(Ala); position 234: Lys(Arg); position 243: Asp(Pro); position 249: Ala(Gly); position 290: Arg(Gly); position 302: Thr(Lys); position 319: Arg(His); position 350: Gly(Val); position 350: Glu(Val); position 395: Lys(Arg); position 402: Asp(Glu); position 419: Lys(Arg).

Therefore, preferred S-GT proteins of the invention include variants of the protein of SEQ ID No. 28 having at least one of the above amino acids in the protein of SEQ ID No. 28 changed into another amino acid indicated in the above list.

Preferably, Cysteine amino acids are not altered in S-GT enzymes having substantial sequence similarity to the S-GT form encoded by the cDNA contained in clone pGL9, since SH-bonds are believed to be involved in enzymatic activity. Also, preferred s-gt DNA's or coding regions in accordance with this invention are those DNA sequences encoding the S-GT variants, having at least one of the above amino acid substitutions. A functionally equivalent variant or part of the S-GT enzyme of SEQ ID No:28, in accordance with this invention, is an enzyme having at least 80 %, preferably at least 90 %, of the activity of this S-GT enzyme (SEQ ID No:28) using the glucosyltransferase radioassay of GrootWassink et al. (1994b, supra), when analyzed under the same test conditions.

Furthermore, given the DNA sequence of the isolated S-GT form, variants can be designed having a different codon usage or containing additional untranslated sequences such as introns but encoding the same protein or a protein having substantial sequence similarity. Also, based on the amino acid sequence of the protein encoded by the cDNA comprised in clone pGL9, variants can be made differing in some amino acids but having substantially the same glucosyltransferase activity. Upon initial comparison of the protein primary structure with other plant glucosyltransferases, particularly the protein parts from amino acids 16-42, 180-185, 346-375, 448-453 in SEQ ID No. 28 should be preferentially retained when designing variants of the B. napus S-GT enzyme. Therefore, amino acid substitutions in these regions should be kept to minimal (e.g., no more than 10 %, preferably no more than 5 %) in order to retain most of the S-GT activity.

In a preferred embodiment of this invention, DNA sequences with substantial sequence similarity to the s-gt DNA of the invention include DNA sequences encoding the protein produced by plasmid pGL9 contained in E. coli WK6, deposited in accordance with the Budapest Treaty at the BCCM-LMBP on September 7, 1995 under accession number 3344.

Variants of the S-GT enzyme can be isolated based on the knowledge of the protein peptidic fragments of the B. oleraceae S-GT enzyme of the invention. A S-GT enzyme variant of the present invention should retain the enzyme's catalytic site and thus are characterized by their clear S-GT activity in the radioassay of Reed et al. (1993, supra) and the presence of a continuous stretch of amino acids with more than 85 % sequence similarity, particularly more than 90 % sequence similarity, preferably more than 95 % sequence similarity, to any one of the peptide fragments 1 to 7 of SEQ ID. Nos. 1 to 7. Preferentially variants of the S-GT enzyme of this invention have S-GT activity and comprise any of the peptides of SEQ ID Nos 1 to 7, preferably the peptide of SEQ ID No. 1, in their primary sequence. Since it was found that none of the peptides of SEQ ID Nos 1 to 7 are found in any known protein, the presence of any of these peptides in a protein sequence having glucosyltransferase activity characterizes the S-GT protein of the invention.

Generally in accordance with the present invention, s-gt inhibitory chimeric genes are provided for plant transformation. As used herein, "s-gt inhibitory chimeric gene" is a chimeric gene comprising all or part of a coding sequence encoding an RNA or protein inhibiting s-gt gene expression. For example, a chimeric gene of the present invention includes all or part of a DNA encoding an antisense RNA, a sense RNA, or a ribozyme, inhibiting s-gt gene expression when produced in a plant cell. The specific coding region of an s-gt inhibitory chimeric gene typically also comprises, besides a coding region, a promoter region, a coding region, and a 3' transcription termination and polyadenylation region.

Any plant expressible promoter can be used to express the inhibitory chimeric gene of the present invention.

Preferred plant-expressible promoters of the s-gt inhibitory chimeric gene of this invention include, but are not limited to : the strong (constitutive) 35S promoters (the "35S promoters") of the cauliflower mosaic virus of isolates CM 1841 (Gardner et al., 1981, Nucleic Acids Research 9, 2871-2887), CabbB-S (Franck et al., 1980, Cell 21, 285-294) and CabbB-JI (Hull and Howell, 1987, Virology 86, 482-493); the ubiquitin promoter (EP 0342926), and the TR1' promoter and the TR2' promoter which drive the expression of the 1' and 2' genes, respectively, of the Agrobacterium tumefaciens T-DNA (Velten et al., 1984, EMBO J. 3, 2723-2730).

Alternatively, a promoter can be utilized which is not constitutive but rather is specific for one or more tissues or organs of the plant, such as the pod tissue, whereby the inserted chimeric s-gt inhibitory gene or its functionally effective part is expressed only in cells of the specific tissue or organ. Since it has been shown that the important glucosinolates stored in the seed are derived from the adjacent pod tissue, particularly from the pod walls (Toroser et al, 1995, Annual Meeting of the American Society of Plant Physiologists, Charlotte, North Carolina, July 29-August 2, USA, in Supplement to Plant Physiology, vol. 108, abstract 716), a plant-expressible promoter can be utilized which is at least expressed in pod tissue, and particularly in the pod wall; preferably highly expressed in pod tissue, more preferably the pod wall. Pod tissue-specific promoters as used here include the promoters of the genes encoding pod-specific mRNAs, e.g., the promoters of the genes encoding the mRNAs reported by Coupe et al. (1993, Plant Mol. Biol. 23, 1223; 1994, Plant Mol. Biol. 24, 223).

For example, preferential expression in the pod tissue by a pod tissue-specific promoter, preferably in the pod wall (e.g., the carpels constituting the pod wall) by a pod-wall-specific promoter is an alternative method to lower seed glucosinolate levels without interfering, to a large extent, with the glucosinolate content of the leaves and other tissues, which may be more desirable in certain circumstances.

"Pod tissue", as used herein, refers to the cells constituting a pod, including structures such as the carpels forming the pod wall, the (false) septum, and the replum but excluding the seeds.

In yet another embodiment of the present invention, the promoter for the s-gt inhibitory chimeric gene of the invention can also be the promoter of the endogenous s-gt gene, the mRNA of which corresponds to the cDNA contained in plasmid pGL9, or can be a leaf-specific, stem-specific and in some cases even an embryo- or seed-specific promoter.

In accordance with this invention, the s-gt inhibitory chimeric gene, or a functionally effective part thereof, is inserted in the plant genome such that the inserted coding region is upstream (i.e., 5') of suitable 3' transcription termination and polyadenylation signals (i.e., transcript termination and polyadenylation signals). Preferred polyadenylation and transcript formation signals include but are not limited to those of the 35S gene (Mogen et al., 1990, The Plant Cell 2, 1261-1272), the octopine synthase gene (Gielen et al., 1984, EMBO J 3, 835-845) and the T-DNA gene 7 (Velten and Schell, 1985, Nucl. Acids Res. 13, 6981-6998), which act as 3'-untranslated DNA sequences in transformed plant cells. Alternatively, the 3' transcription termination and polyadenylation signals can be obtained from a pod tissue or pod wall specific gene or from a plant s-gt gene.

More specifically, the coding region of the s-gt inhibitory chimeric gene of the invention comprises a DNA encoding an RNA or protein interfering with the normal expression of the s-gt gene in the plant cell. A preferred coding region in accordance with this invention comprises a DNA sequence encoding an s-gt antisense RNA, or functionally effective variants thereof. In accordance with this invention, "s-gt antisense RNA" is an RNA complementary to at least part, preferably a functionally effective part, of the RNA, particularly the mRNA, encoded by an endogenous plant s-gt gene. For example, an RNA complementary to a region of at least 100 nucleotides, preferably a region of at least 500 nucleotides, of the (m)RNA encoded by the s-gt gene, the cDNA of which is contained in pGL9 (deposited at the BCCM-LMBP under number LMBP 3344) can be utilized. An RNA complementary to a region of at least 100 nucleotides, preferably a region of at least 500 nucleotides, of the (m)RNA encoded by the s-gt gene, the cDNA of which has the sequence illustrated in SEQ ID No. 28 is encompassed by the present invention. Similarly, as described above for the s-gt DNA sequence, variants of the s-gt antisense RNA can be made, provided they have sufficient complementarity, more preferably at least 90 % complementarity, particularly at least 95 % complementarity, to the s-gt RNA formed in the plant cell.

Surprisingly, it was found that there are several isoform DNAs of the invention which have substantial sequence similarity, so one s-gt antisense RNA will be able to inhibit expression of all isoform genes. The antisense technology is based on blocking the information flow (by transcription and translation) from DNA to RNA and/or protein by the introduction of an RNA strand ("antisense RNA") complementary to the sequence of an endogenous target ("sense") RNA (Murray & Crockett, 1992, in Antisense RNA and DNA, pp. 1-50, Murray, JAH (ed.), Wiley-Liss, New York). The outcome of this is a partial to complete silencing of endogenous gene expression (see, e.g., EP 0 467 349; EP 0 223 399; EP 0 240 208).

It has been shown that even the most abundant protein present in plants can be reduced effectively using antisense techniques (Rodermel et al., 1988, Cell 55, 673; Jiang et al., 1994, Plant Mol. Biol. 25, 569-576). The antisense RNA can be complementary to only part of the (m)RNA transcribed from the gene that is to be inactivated. Preferably, such a part is at least about 100 basepairs long, more preferably at least about 500 basepairs long, typically between about 500-1000 basepairs long. Also, an antisense RNA which is complementary to the full (m)RNA to be inactivated, or to only the 5' and 3' untranslated regions, can be used.

In view of the down-regulation desired, the antisense RNA can be complementary to certain stretches of conserved sequences or to the most divergent sequences between the different s-gt RNA forms. Hence, the expression of a specific s-gt isoform, or of several isoforms sharing a conserved region, can also be inhibited. For isoform-specific inactivation of the s-gt gene, the 5' leader and 3' trailer sequences will be more useful target sequences, since it has been shown in the present invention that the S-GT isoform DNAs differ most in these regions, while differences in the coding region are limited. When different antisense RNAs can be used, it is preferred to use the antisense RNA which is most stable in the plant cell, particularly in the nucleus.

A preferred antisense construct of this invention is a construct comprising a DNA sequence encoding an RNA which is complementary, preferably 90% to 100% complementary, to at least 100 nucleotides of the RNA encoded by the s-gt gene, the mRNA of which corresponds to the cDNA sequence contained in plasmid pGL9 (deposited in host organism E. coli WK6 under accession number BCCM-LMBP 3344). It is more preferable to utilize a DNA sequence encoding an RNA which is complementary, preferably 90% to 100% complementary, to at least 100 nucleotides up to the entire mRNA sequence encoded by the s-gt gene, wherein a cDNA prepared from this mRNA has the sequence shown in SEQ. ID No. 28, or has substantial sequence similarity to the sequence of SEQ ID No. 28.

Preferred DNA sequences encoding an antisense s-gt RNA, in accordance with this invention, include DNA sequences encoding those RNAs hybridizing under stringent conditions with the (m)RNA (or parts thereof) encoded by the s-gt gene in a plant cell. This antisense RNA is at least 80 %, more particularly at least 90 %, more preferably at least 95 % complementary to the s-gt (m)RNA, particularly the s-gt mRNA, the cDNA of which is contained in plasmid pGL9 (BCCM-LMBP 3344). Indeed, it has been shown that for antisense inhibition of gene expression, 100 % complementarity is not required, however the antisense RNA has to be sufficiently complementary to the sense RNA so that they can readily hybridize under stringent conditions. This way, an entire gene family having high sequence similarity can be inhibited by expressing one antisense RNA which is 100 % complementary to one of the members of the gene family (see, e.g., Rodermel et al., 1988, supra).

In a further embodiment of this invention, the coding region of the chimeric s-gt inhibitory gene encodes a sense RNA which is identical, preferably has more than 80 % sequence similarity, more preferably has more than 90 % sequence similarity, most preferably has at least 95 % sequence similarity, to at least part of the (m)RNA transcribed from the gene, the cDNA of which corresponds to the DNA sequence contained in plasmid pGL9, preferably the cDNA with the sequence of SEQ ID No. 28, or functionally effective parts thereof. Similarly as for the antisense approach, a 5' or 3' part of the coding region, the full coding region as well as the 5' or 3' untranslated regions or the intron sequences can be used for gene silencing. Although antisense suppression is the preferred embodiment of this invention, the sense suppression (or cosuppression) mechanism (Flavell et al., 1994, Proc. Natl. Acad. Sci. USA 91, 3490-3496) can also yield plants having significant inhibition of s-gt gene expression in selected transformants.

Alternatively, the coding region of the chimeric s-gt inhibitory gene comprises a DNA sequence encoding a catalytic RNA molecule called a ribozyme (PCT publication WO 89/05852; Nature 334, 585-591, 1988), comprising a targetting region which is complementary, preferably 90% to 100% complementary, to part of the RNA formed by an s-gt gene, the mRNA of which corresponds to the cDNA contained in plasmid pGL9, or functionally effective variants thereof. The use of ribozymes allows the targetting of conserved or divergent RNA sequences, such that the expression of a group of S-GT isoforms (sharing a conserved region) or a particular S-GT isoform can be inhibited. Similarly, the EGS technology (e.g., PCT patent publication WO 93/22434) can be used to specifically inactivate s-gt gene expression.

The chimeric s-gt inhibitory gene can also encode an RNA encoding a protein inhibiting S-GT activity, such as an antibody fragment specifically binding to an S-GT, particularly the S-GT encoded by the DNA sequence comprised in clone pGL9, contained in E. coli WK6 (BCCM-LMBP 3344), more preferably the S-GT with the amino acid sequence of SEQ ID No. 28. Such antibodies or the Fab fragments thereof with high affinity to the S-GT protein isolated from clone pGL9 can be expressed in plants (Tavladoraki et al., 1993, Nature 366, 469-472), so that the endogenous S-GT protein is rendered inactive.

Since the S-GT enzyme was found to be non-specific for the side chain of the glucosinolate produced (Jain et al., 1988, J. Plant Physiol. 136, 356-361) the concentrations of all glucosinolates, and not just one type, normally produced in the plant, will be lowered when expressing a chimeric s-gt inhibitory gene, preferably a DNA sequence encoding an s-gt antisense RNA, in plants.

In order to transfer all or a functionally effective part of a chimeric s-gt inhibitory gene to a plant cell genome, suitable restriction sites can be introduced, flanking the chimeric gene or its part. This can be done by site-directed mutagenesis, using well-known procedures (e.g., Stanssens et al., 1989, Nucl. Acids Res. 12, 4441-4454; White et al., 1989, Trends in Genet. 5, 185-189).

In another embodiment of the invention, plant cells are transformed with the above chimeric genes, and plants are regenerated from such transformed cells. A disarmed Ti plasmid, containing the chimeric s-gt inhibitory gene, in Agrobacterium tumefaciens can be used to transform the plant cell, and thereafter, a transformed plant can be regenerated from the transformed plant cell using the procedures described, for example, in EP 0116718, EP 0270822, PCT publication WO 84/02913 and EP 0242246 (which are also incorporated herein by reference), and in Gould et al. (1991, Plant Physiol. 95, 426-434), or the method described in PCT publication WO 94/00977. Of course, other types of vectors can be used to transform the plant cell, using procedures such as direct gene transfer (as described, for example, in EP 0233247), pollen mediated transformation (as described, for example, in EP 0270356, PCT publication WO 85/01856, and US Patent 4,684,611), plant RNA virus-mediated transformation (as described, for example, in EP 0067553 and US Patent 4,407,956), and liposome-mediated transformation (as described, for example, in US Patent 4,536,475).

A resulting transformed plant, such as a transformed oilseed rape plant, can be used in a conventional plant breeding scheme to produce more transformed plants with the same characteristics or to introduce the chimeric s-gt inhibitory gene in other varieties of the same or related plant species or into commercial hybrid plants. Seeds, which are obtained from the transformed plants, contain the chimeric s-gt inhibitory gem as a stable genomic insert.

The preferred plants to be transformed in accordance with this invention include but are not limited to Crucifer plants, particularly Brassica plants, preferably oilseed rape Brassica plants, more preferably Brassica napus plants. The plants of the invention are characterized by their significantly lower levels of expression of the endogenous s-gt gene, preferably these plants are characterized by the lower glucosinolate content in their tissues, particularly in their seeds.

Hybrid plants, in accordance with this invention, can be made by crossing a transformed plant of the invention, homozygous for the s-gt inhibitory gene, with a male sterile plant, wherein the male sterile plant preferably is obtained by following the method described in EP 344029 and by Mariani et al. (1990, Nature 347, 737-741; 1992, Nature 357, 384-387).

In yet another preferred embodiment of the invention, the obtained hybrid plants containing the chimeric s-gt inhibitory gene correspond to the "canola" definition set out by the Canola Council of Canada. For oilseed rape to be designated "canola", oil obtained after crushing the seed has to contain less than 2 % of total fatty acids in the oil as erucic acid and less than 30 µmoles of alkenyl glucosinolates per gram of dry matter of the (oil-free) seed meal. Therefore, the eventual hybrid plants obtained in accordance with this invention preferably have a content of alkenyl glucosinolates, preferably total glucosinolates, of less than 30 µmoles, preferably less than 15 µmoles, more preferably less than 5 µmoles per gram defatted dry matter of the seed. In the most preferred embodiment of this invention, the plants contain less than 0.5 µmoles total glucosinolates, preferably alkenyl glucosinolates, per gram dry matter of defatted seed, when the plants are hemizygous for the introduced chimeric s-gt inhibitory gene.

In yet another preferred embodiment of this invention, those tissues or organs of the plant other than pods or seeds can still contain their "wild-type" glucosinolate content. However, the total glucosinolate content, preferably the alkenyl glucosinolate content, in the entire plant is reduced to the above cited levels, preferably to undetectable levels.

Tentatively, promoters specific for leaves or other plant tissues or organs can be used to lower the glucosinolate content in these specific tissues or organs while maintaining the "wild-type" glucosinate levels in other parts of the plant.

In a preferred embodiment of this invention, the glucosinolate content in the plants, preferably in the seeds, is significantly decreased in hybrids, obtained from cross-fertilization of two parent plants, at least one of which has been transformed to inhibit s-gt gene expression in accordance with this invention. Further in accordance with this invention, the total glucosinolate levels in the seed of a hybrid Brassica plant, obtained in accordance with the teachings of the present invention, both parents of which contain less than 30 µmoles of alkenyl glucosinolates per gram dry defatted seed matter, is reduced. Preferably, hybrid plants are provided, wherein at least one of the parents has total glucosinolate levels of more than 30 µmoles, preferably more than 50 µmoles, per gram dry defatted seed and contains less than 30 µmoles of alkenyl glucosinolates per gram of defatted seed matter. Even more preferably, hybrid plants are provided, wherein at least one of the parents has total glucosinolate levels of more than 5 µmoles, preferably more than 50 µmoles, per gram in the defatted seed meal, and contains total glucosinolate levels in the whole seed basis of less than 5 µmoles per gram. Most preferably, hybrid plants are provided, from which at least one of the parents has glucosinolate levels above 0 µmoles (is the background levels found in the controls), preferably above 50 µmoles, and has no detectable glucosinolates in the seeds (0 µmoles).

In a further embodiment of this invention, a process is provided for inhibiting expression of an s-gt gene in a plant cells, particularly a process for lowering glucosinolate levels in plants, particularly Brassica plants. This process comprises the steps of transforming plant cells, preferably Brassica plant cells, with any of the above chimeric inhibitory s-gt genes, and then regenerating a plant from these transformed cells.

In yet another preferred process of the invention, plant cells are transformed with a DNA sequence encoding an antisense RNA complementary to at least part of the (m)RNA transcribed from the s-gt gene, particularly a DNA sequence encoding an antisense RNA complementary to the RNA, the cDNA of which is contained in plasmid pGL9; more particularly a DNA sequence encoding an mRNA, the cDNA of which comprises the sequence of SEQ ID No. 28 or a DNA sequence having substantial sequence similarity thereto.

Several assays are available for measuring both total and individual glucosinolates in plants or parts thereof (e.g., Quinsac and Ribailler, 1991, Assoc. Off. Anal. Chem. 74, 932-939; Reed et al., 1993, supra). Depending on the desired characteristics of the obtained plants, total glucosinolates or only glucosinolates of one type, preferably alkenyl glucosinolates, can be measured so that those plants with a reduced total glucosinolate content or with a reduced content in a certain type of glucosinolates, preferably alkenyl glucosinolates, can be selected after transformation. In accordance with this invention, the concentration of all glucosinolates formed by the S-GT enzyme, particularly the concentration of all alkenyl glucosinolates, is significantly lowered in the plants of the invention, preferentially in their seeds.

Since it is expected that other Cruciferous plants, particularly other Brassicaceae, have similar S-GT enzyme forms, differing in some characteristics but sharing regions with substantial sequence similarity, the antisense, ribozyme or co-suppression approaches outlined above can similarly be applied to lower the glucosinolate content in other Brassica species, preferably oilseed Brassica species. Preferred Brassicaceae to be transformed in accordance with this invention to inhibit s-gt expression, preferably to decrease glucosinolate content in the seed, besides Brassica napus, include Brassica juncea, Brassica oleraceae, Brassica carinata, Brassica nigra, Brassica campestris and the like, and any intergenic crosses or synthetic varieties thereof.

In yet another preferred embodiment of this invention, the chimeric s-gt inhibitory gene is designed such that it effectively inhibits S-GT isoforms of both B. napus and B. juncea, so that glucosinolate production by genes of both parent genomes of these amphidiploid species is inhibited.

The thus obtained plants with lowered glucosinolate content, particularly in their seeds, are then used to cross into elite allele lines, preferably hybrid plants. Therefore, the effect of glucosinolate-reduction should preferably be apparent in the hemizygous state. In certain cases, two parent plants, transformed in accordance with this invention to have a lower glucosinolate content, particularly in their seeds, can be crossed to form a hybrid having a further reduced glucosinolate content. The following Examples are offered by way of illustration and not by way of limitation. The sequence listing referred to in the Examples is as follows:
- SEQ ID No. 1:: B. oleraceae S-GT peptide fragment 1
- SEQ ID No. 2:: B. oleraceae S-GT peptide fragment 2
- SEQ ID No. 3:: B. oleraceae S-GT peptide fragment 3
- SEQ ID No. 4:: B. oleraceae S-GT peptide fragment 4
- SEQ ID No. 5:: B. oleraceae S-GT peptide fragment 5
- SEQ ID No. 6:: B. oleraceae S-GT peptide fragment 6
- SEQ ID No. 7:: B. oleraceae S-GT peptide fragment 7
- SEQ ID No. 8:: primer gl3
- SEQ ID No. 9:: primer gl7
- SEQ ID No. 10:: 3' RACE oligo(dT) CDS primer sequence
- SEQ ID No. 11:: 3' RACE Anchor primer sequence
- SEQ ID No. 12:: primer gl5
- SEQ ID No. 13:: primer gl9
- SEQ ID No. 14:: sequence of cDNA clone pGL2-7 (incorporated primer regions have not been added, part of polyA tail is shown)
- SEQ ID No. 15:: sequence of cDNA clone pGL2-25 (incorporated primer regions have not been added, part of polyA tail is shown)
- SEQ ID No. 16:: primer gl1
- SEQ ID No. 17:: primer glP1
- SEQ ID No. 18:: primer glP2
- SEQ ID No. 19:: sequence of cDNA clone pGL3-22 (incorporated primer regions have not been added)
- SEQ ID No. 20:: sequence of cDNA clone pGL4-2 (incorporated primer regions have not been added)
- SEQ ID No. 21:: primer glP4
- SEQ ID No. 22:: primer glP5
- SEQ ID No. 23:: sequence of 5' RACE Anchor (Clontech)
- SEQ ID No. 24:: sequence of 5' RACE Anchor primer (Clontech)
- SEQ ID No. 25:: sequence of cDNA clone pGL6-14 (incorporated primer regions have not been added)
- SEQ ID No. 26:: primer gl31
- SEQ ID No. 27:: primer gl32
- SEQ ID No. 28:: cDNA sequence of the pGL9 clone 22 (primer regions have been included), and corresponding translated protein
- SEQ ID No. 29:: amino acid sequence of the protein produced by pGL9 clone 22

Unless otherwise stated in the Examples, all procedures for making and manipulating recombinant DNA are carried out by the standardized procedures as described in volumes 1 and 2 of Ausubel et al., Current Protocols in Molecular Biology, Current Protocols, USA (1994) and Sambrook et al., Molecular Cloning - A Laboratory Manual. Second Ed., Cold Spring Harbor Laboratory Press, NY (1989). Standard methods and materials for plant molecular biology work are described in Plant Molecular Biology LABFAX, edited by R.R.D. Croy (1993, Bios Scientific Publishers and Blackwell Scientific publications, series editors B.D. Hames and D. Rickwood).

### EXAMPLES

### Example 1

### B. oleraceae S-GT protein identification

The S-GT protein of B. oleraceae spp. botrytis was isolated as described by GrootWassink et al. (1994b, supra) which is incorporated herein by reference. The partial amino acid sequence of the isolated thiohydroximate S-glucosyltransferase was determined. Since the N-terminus was found to be blocked, the purified protein was first subjected to partial tryptic digestion. The following internal peptide fragments were obtained:
1) Val-Thr-Ile-Ala-Thr-Thr-Thr-Tyr-Thr-Ala-Ser-Ser-Ile-Ser-Thr-Pro-Ser-Val-Ser-Val-Glu-Pro-Ile-Ser-Asp-Gly-His-Asp-Phe-Ile-Pro (SEQ ID No. 1)
2) Ala-Leu-Gln-Gln-Ser-Asn-Phe-Asn-Phe-Leu-Trp-Val-Ile-Lys (SEQ ID No. 2)
3) Gly-His-Val-Val-Val-Leu-Pro-Tyr-Pro-Val-Gln-Gly-His-Leu-Asn-Pro-Met-Val-Gln-Phe-Ala-Lys (SEQ ID No. 3)
4) Ala-Thr-Leu-Ile-Gly-Pro-Met-Ile-Asp-Ser-Ala-Tyr-Leu-Asp-Lys (SEQ ID No. 4)
5) Leu-Pro-Glu-Gly-Phe-Val-Glu-Ala-Thr-Lys (SEQ ID No. 5)
6) Phe-Val-Glu-Glu-Val-Trp-Lys (SEQ ID No. 6)
7) Ala-Met-Ser-Glu-Gly-Gly-Ser-Ser-Asp-Arg-Ser-Ile-Asn-Glu-Phe-Val-Glu-Ser-Leu-Gly-Lys (SEQ ID No. 7)

These fragments represent about 1/4 of the B. oleraceae S-GT protein sequence and unambiguously characterize the S-GT enzyme.

Searches in protein sequence databases showed that the protein characterized by the above peptide fragments 1 to 7 is unique and has no significant sequence similarity with any previously known protein. Also, each of the peptide fragments 1 to 7 were not found to be included in any known protein. A peptide fragment of a protein showing 85 % sequence similarity with the short fragment 6 was found to be the closest match. Thus, each of the fragments identified above specifically identifies the isolated S-GT protein of B. oleraceae.

### Example 2

### Isolation of a B. napus s-gt gene

Total RNA was prepared from B. napus cv. Westar seedlings that germinated for 5 days in the dark following routine extraction techniques. About 1 mg RNA per gram tissue was obtained as was established by OD₂₆₀ measurements. RNA quality was checked on a 2% TBE agarose gel (under RNase free conditions) for absence of high molecular weight (MW) DNA and integrity. mRNA was prepared from 1 mg of total RNA using the Promega kit "PolyAttract mRNA Isolation System IV" as described by the suppliers. Approximately 2-4 µg of mRNA was recovered.

From the 7 peptide sequences derived from the purified B. oleraceae S-GT protein (SEQ ID Nos 1-7), 7 sets of complementary degenerated primers (of which 2 were nested sets) were selected and synthesized by conventional methods. PCR-RACE (rapid amplifying of cDNA ends) with combining degenerate primers (based on the B. oleraceae peptide fragments) and the 3' Anchor primer of the 3' RACE kit (Clontech) was used directly. Combinations of these primer sets were tried in PCR reactions on first strand cDNA made with the Clontech kit "3'AmpliFINDER™ RACE Kit" following the instructions of the supplier. This 3'RACE cDNA was used in PCR reactions where primers gl3 (SEQ ID No. 8) or gl7 (SEQ ID No. 9) were combined with the Anchor Primer of the Clontech 3'RACE kit (SEQ ID No. 11; complementary primer to the NN₁ oligo (dT)₁₈ CDS Primer (shown in SEQ ID No. 10)). Both PCR reactions were used as a template for a second semi-nested PCR with anchor primer and respectively primer gl5 (SEQ ID No. 12) or gl9 (SEQ ID No. 13). In the first case (for the gl3-gl5 combination) a PCR fragment of approximately 650bp was amplified. Upon A/T cloning of this fragment in the vector pGEM-T (Promega) following the instructions of the suppliers and electrotransformation in E.coli SURE (Stratagene) cells, two of the resulting transformants (pGL2 clones 7 and 25) having the appropriate insert size were sequenced. Analysis of the sequences showed an open reading frame of about 470bp, a 104bp 3'UTR and the polyA⁺tail for both clones. The sequence determined for pGL2-7 is shown in SEQ ID No. 14, that for pGL2-25 in SEQ ID No. 15. The amino acid sequence of the protein fragment encoded by the open reading frame contained in both these clones revealed part of the S-GT peptide 2 (as expected because this sequence was used for PCR-cloning), and the complete S-GT peptides 5, 6 (with one amino acid difference) and 7.

Using the cDNA obtained from the mRNA pool with the 5' RACE kit (Clontech) using the glP1 primer (SEQ ID No. 17) in a PCR amplification in which the degenerated primers gl1 and gl7 (SEQ ID Nos 16 and 9, respectively) were used in combination with glP2 (SEQ ID No. 18) resulted in the generation of specific PCR products of respectively 850bp or 1000bp in length. These two PCR fragments were A/T cloned in the Promega pGEM-T vector as described above and named respectively pGL4-2 and pGL3-22. The PCR product in pGL4 was completely contained in that of pGL3. pGL3 includes 982bp of s-gt ORF, of which the last 116bp overlap the s-gt fragment cloned in pGL2. The sequence of pGL3-22 is shown in SEQ ID No. 19, that of pGL4-2 in SEQ ID No. 20.

In addition to the S-GT peptide sequences 2, 5, 6 and 7 previously found in pGL2 the remaining peptide sequences 1, 3 (partially) and 4 (with one amino acid difference) were now located.

In order to clone the remaining 5'end of the B.napus s-gt gene 5'RACE was used, with a new set of nested gene specific primers located at the 5' end of the pGL3 PCR insert, namely glP4 and glP5 (SEQ ID Nos 21 and 22, respectively). The Clontech 5' AmpliFINDER™ RACE Kit was used according to the supplier's recommendations. The first strand cDNA was generated by reverse transcriptase using primer glP4, and ligated to the 5' RACE AmpliFINDER™ Anchor (SEQ ID No. 23). Upon amplification of this first strand with the kit's AmpliFlNDER™ anchor primer (SEQ ID No. 24) and the gene specific primer glP5 a 300bp PCR fragment was generated. After A/T cloning (see above) and transformation in E.coli WK6 with blue-white screening, several recombinant clones were sequenced. One of them, pGL6 clone14 (pGL6-14), had the desired 5' end of the s-gt gene. The sequence showed an 88bp 5'UTR and a partial ORF of 163bp, including the full DNA sequence of S-GT peptide 3 and the start of S-GT peptide 1 (SEQ ID No. 25).

Based on the sequences of the s-gt cDNA fragments retrieved in the previous steps the forward primer gl31 (5'-TTA TTT TTC TTC TTC CTC CTC CTC T-3', SEQ ID No. 26) and reverse primer gl32 (5'-AGC AAC AAC AAC AAA CAC ACA AGA T-3', SEQ ID No. 27) were designed, located respectively in the 5'UTR and the 3'UTR. These two primers were used in PCR reactions on double-stranded (ds) cDNA of B. napus cv. Westar (Superscript Lambda System for cDNA Synthesis and -cloning, BRL, cat.no 8256RT). cDNA was amplified using 0.2 µM of these two primers in a reaction buffer consisting of 20 mM Tris-HCl (pH8.4), 50 mM KCl, 2 mM NaCl, 0.2 µM (each)dNTP-mixture and 2.5 Units of Taq-DNA polymerase (purchased at Gibco-BRL). Amplification conditions consisted of a initial 5 minutes denaturing at 94°C, 30 cycles of 45 sec 94°C denaturing, 60°C annealing for 45 sec, and 3 min of elongation at 72°C. As expected, an amplification product of 1.5 kb was obtained. This DNA product was isolated and cloned in the pGEM-Z vector of Promega by A/T-cloning. One clone was retrieved, namely pGL9 clone 22 (further referred to as "the pGL9 clone"). The sequence of this clone revealed an s-gt cDNA comprising the complete open reading frame (SEQ ID No. 28 shows the full sequence obtained; the regions corresponding to the primers gl31 and gl32 are included at the 5' and 3' end, respectively (note that the region corresponding to primer gl31 is partly deleted during cloning)), with a sequence similarity highest to pGL2-25 and pGL4-2. This cDNA comprises an open reading frame encoding a protein with a calculated m.w. of about 51 kD (50,901). The differences between the coding regions of pGL9-22 and the coding regions comprised in the partial cDNA clones obtained above (pGL2, pGL3, pGL4, and pGL6) are illustrated in Figure 2. This Figure confirms the high degree of sequence similarity between the isolated cDNA fragments. Figure 1 shows the approximate locations of the different cDNA fragments in comparison to the pGL9 cDNA clone, and the primers used in their isolation.

The cDNA clone pGL9-22, contained in the pGEM5Zf(+) vector, has been deposited in E. coli strain WK6 at the Belgian Coordinated Collections of Microorganisms (BCCM) - Laboratory for Molecular Biology - Plasmid collection (LMBP) on September 7, 1995 under accession number BCCM-LMBP 3344.

Another full length s-gt cDNA clone was obtained using the Clontech Marathon cDNA Amplification Kit. Here mRNA, isolated using the methods and kits mentioned before, was converted to double-stranded cDNA following the provided protocol. In the following amplification reaction on this material, primers gl31 and gl32 were used in combination with several heat stable DNA polymerases, such as Taq DNA polymerase (Gibco,BRL), Pwol DNA polymerase (Boehringer), Vent DNA polymerase (New England Biolabs, Tth,XL DNA polymerase (Perkin Elmer Cetus, a commercial mixture of Tth DNA polymerase with a small amount of Vent DNA polymerase). Reaction mixtures were all made following the purchasers recommendations for optimizing conditions. Cycling parameters after the initial 5′94°C denaturation step were 30 cycles either of 45˝94°C,3′68°C or 45˝94°C,45˝60°C,2′72°C. After a second round of PCR using 1 µl of a tenfold dilution of the previous PCR, both Taq DNA polymerase (conditions as mentioned above) and Tth,XL DNA polymerase (1XTth,XL DNA polymerase buffer supplemented with 1.0 mM Mg(OAc)2) showed the expected 1.5kb amplification product. The Tth,XL DNA polymerase reaction was done using as primers gl31 and gl32 where respectively PstI and XbaI restriction sites were attached at the 5'end and 3' end. Following XbaI-PstI digestion, the PCR amplified DNA fragment was cloned in pUC19. E.coli MC1060 transformation gave approximately 10⁸ transformants/µg transforming vector all having the expected insert, which was named pGL18. Partial DNA sequencing of fragments (1 fragment of 200 to 300 basepairs) of the pGL18 clone 1 shows that the pGL18-1 clone is identical to the corresponding parts in the pGL9 clone (see, e.g., Fig. 2).

The coding region of the pGL9 cDNA clone was cloned in the PstI-XbaI sites of the polylinker of the pUC19 expression vector using conventional procedures, so that it is under the control of the lac promoter. Expression of this chimeric gene in E. coli by IPTG induction showed a band recognized by rabbit polyclonal anti-B. oleraceae S-GT antibody, while this band was not present in the untransformed induced strain, upon Western blotting. The recombinantly produced S-GT protein was confirmed to have a glucosyl-S-transferase activity in the radioassay of GrootWassink et al. (1994b, supra). This assay was based on the incorporation of [14C]Glc from UDP-Glc into phenylacetothiohydroximate. As starting material, the above E. coli strains containing the pGL14 clone were used. The wild type WK6 E. coli strain was used as a control, as well as water. Strains were grown and induced (with IPTG) overnight. A Western blot and a Coomassie staining were first performed to check for expression of the formed fusion protein. The protein concentration was assessed by a Bradford assay, using BSA as a standard. For the glucosinolate assay, 80 µl of reaction mixture (50 mM Mes buffer with UDP-[U^{14C}]Glc (0.05 µCi) and 1.0 mM phenyl acetothiohydroximate, 5 mM MgSo₄ and 0.1% ME) was mixed with 20 µl of extract (from 1 ml of overnight culture that went 3 times through the French press, 800 Pa/inch²; in a 1/100 or 1/500 dilution, depending on the protein concentration of each of the two samples). The samples were incubated at 30°C in a water bath and the reaction was stopped by boiling for 10 min. After cooling on ice, 500 µl ethylacetate was added and the tube containing the samples were vigorously skaken. Samples were spun down and the top phase (containing the glucosinolate in ethylacetate) was recovered for radioactivity measurement.

The results showed that, at the proper dilution of the cell extract (between 1/100 and 1/500 depending on the sample), the enzyme activity increased with time and was significantly higher than the controls. Using the 0 min reaction as a control, the following relative enzyme activities were obtained after different time periods: 22 after 10 minutes (i.e., the glucosyltransferase activity after 10 minutes of incubation with the reaction mixture was 22 times that of the activity of the recombinant s-gt strain at time 0), 30 after 20 min, 40 after 30 min and 80 after 60 min.

Using the wild type E. coli strain as a control, the following relative enzyme activities were observed for the S-GT-producing clone: 1 after 0 min reaction, 23 after 10 min (i.e., after 10 minutes of reaction, the S-GT producing strain had 23 times the glucosyl-transferase activity of the wild type strain), 16 after 20 min, and 65 after 30 min. Thus, these radioassays show that the s-gt gene cloned in the transformed E. coli WK6 produced a protein with a significant S-GT activity, thus confirming that the correct DNA sequence had been isolated. The S-GT proteins encoded by the full open beading frames of the other isoforms corresponding to the cDNA fragments isolated above show the same significant S-GT activity under similar assay conditions.

In a next step, the complete Brassica napus s-gt gene is cloned from a genomic DNA library using the above sequence information. The promoter region of the s-gt gene is identified in the genomic clone corresponding to the cDNA contained in plasmid pGL9 (as deposited under deposit number BCCM-LMBP 3344). The promoter region, consisting of a DNA sequence comprising 1000 bp upstream of the open reading frame, is isolated for further construction work.

### Example 3:

### Antisense inhibition of s-gt gene expression in oilseed rape plants

Using standard plant molecular biology techniques, several constructs based on the above isolated full cDNA (pGL9, SEQ ID No. 28) are inserted into oilseed rape plant cells which are then regenerated into plants. A B. napus variety having more than 30 µmoles of alkenyl glucosinolate per gram oil free seed matter is transformed with the s-gt antisense constructs of Example 3. Hypocotyl explants of *Brassica napus* are obtained, cultured and transformed essentially as described by De Block et al. (1989, Plant Physiol. 91, 694), except for the following modifications:
- hypocotyl explants are precultured for 3 days in A2 medium [MS, 0.5 g/l Mes (pH5.7), 1.2% glucose, O.5% agarose, 1 mg/l 2,4-D, 0.25 mg/l naphtalene acetic acid (NAA) and 1 mg/l 6-benzylaminopurine (BAP)].
- infection medium A3 is MS, 0.5 g/l Mes (pH 5.7), 1.2% glucose, 0.1 mg/l NAA, 0.75 mg/l BAP and 0.01 mg/l giberellinic acid (GA3).
- selection medium A5 is MS, 0.5 g/l Mes (pH 5.7), 1.2% glucose, 40 mg/l adenine.SO₄, O.5 g/l polyvinylpolypyrrolidine (PVP), O.5% agarose, 0.1 mg/l NAA, 0.75 mg/l BAP, 0.01 mg/l GA3, 250 mg/l carbenicillin, 250 mg/l triacillin, 0.5 mg/l AgNO₃.
- regeneration medium A6 is MS, 0.5 g/l Mes (pH 5.7), 2% sucrose, 40 mg/l adenine.SO₄, 0.5 g/l PVP, 0.5% agarose, 0.0025mg/l BAP and 250 mg/l triacillin.
- healthy shoots are transferred to rooting medium which was A8: 100-130 ml half concentrated MS, 1% sucrose (pH 5.0), 1 mg/l isobutyric acid (IBA), 100 mg/l triacillin added to 300 ml perlite (final pH6.2) in 1 liter vessels (MS stands for Murashige and Skoog medium (Murashige and Skoog, 1962, Physiol. Plant. 15, 473)).

Hypocotyl explants are infected with Agrobacterium tumefaciens strain C58C1Rif® carrying:
- a helper Ti-plasmid pMP90 (Koncz and Schell (1986), Mol. Gen. Genet. 204, 383) or a derivative thereof (such as pGV4000), which is obtained by insertion of a bacterial chloramphenicol resistance gene linked to a 2.5 kb fragment having similarity with the T-DNA vector pGSV8, into pMP90.
- T-DNA vector pGSV8 containing between the T-DNA borders the chimeric s-gt inhibitory gene.

B. napus plants are transformed to contain the following chimeric s-gt inhibitory gene in plant transformation vector pGSV8: a chimeric gene comprising:
- the 35S3 promoter (Hull and Howell, 1987, Virology 86, 482-493) operably linked to one of the following different DNA sequences:
   1) a DNA encoding an antisense RNA complementary to the about 0.7 Kb StyI-XhoI fragment of the s-gt DNA sequence contained in pGL9 (the 5' half of the s-gt coding region), or
   2) a DNA encoding an antisense RNA complementary to the about 0.65 Kb AvaII-AsnI fragment of the s-gt DNA sequence contained in pGL9 (the 3' half of the s-gt coding region), or
   3) a DNA encoding an antisense RNA complementary to the about 1.3 kb StyI-AsnI fragment of the s-gt DNA sequence contained in pGL9 (the almost complete s-gt coding region).

These 3 sequences were each operably linked to the 3' transcript termination and polyadenylation region of the nopaline synthase gene (DePicker et al., 1982, J. Mol. Appl. Genet. 1, 561), thus giving 3 chimeric genes, each comprising a DNA encoding a different antisense RNA.

To select the transformed cells, a chimeric bar gene (De Almeida et al., 1989, Mol. Gen. Genet. 218, 78) conveying resistance to phosphinotricine is included in the transforming DNA. The bar coding region (Thompson et al., 1987, The EMBO J. 6, 2519) is under the control of the promoter of the Arabidopsis thaliana ribulose-1,5-biphosphate carboxylase small subunit 1A gene (Krebbers et al., 1988, Plant Mol. Biol. 11, 745) and is flanked by the 3' transcript termination and polyadenylation region of gene 7 (Velten and Schell, 1985, Nucleic Acids Res. 13, 6981-6999).

The thus obtained vectors pTKV8, 9 and 10, carry the selectable marker gene in two orientations compared to the s-gt antisense DNA chimeric gene, yielding pTKV8a, pTKV8b, pTKV9a, pTKV9b, pTKV10a, and pTKV10b (a in same orientation, b in opposite orientation of transcription). Plasmid pTKV8a, producing an antisense RNA in plant cells complementary to most of the pGL9 s-gt coding region, was deposited in host cell E. coli MC1061 at the BCCM-LMBP on September 7, 1995 under accession number LMBP 3343.

Following similar procedures, the above (antisense) plant transformation constructs are also made with DNA encoding antisense RNA complementary to the RNA corresponding to all or part of the open reading frame corresponding to the pGL6 clone described above. In other transformation steps, Brassica juncea varieties are also transformed with the pGL9-derived antisense constructs pTKV8, pTKV9 and pTKV10, so as to obtain plants with markedly decreased glucosinolate content in their seeds.

### Example 4:

### Plant selection and agronomic evaluation

The transformed oilseed lines of Example 3, having a single copy antisense s-gt chimeric gene and having reduced glucosinolate content in the seeds, are selected upon transformation. These selected transformed plants are rendered homozygous by doubled haploid methodology, and the obtained homozygous lines are then crossed with a male sterile B. napus variety (obtained by following the teachings of EP 344029 and Mariani et al. (1990, Nature 347, 737-741; 1992, Nature 357, 384-387)) to obtain a hybrid having reduced glucosinolate content and being male sterile. The decrease of glucosinolate content in hybrids obtained from crosses of different B. napus varieties with known glucosinolate levels with the obtained selected male sterile hybrid plants allows the quantitative determination of the reduction in glucosinolate content in a hemizygous state in a hybrid plant. A statistically significant reduction (see, for example, Statistical Methods, eds. Snedecor, G.W. and Cochran, Wig, Iowa State, University Press Iowa, USA, 1976) in alkenyl glucosinolate levels is found in the seeds of selected hybrid plants, thus illustrating that the glucosinolate content in Brassica varieties still having high glucosinolate levels in their seeds can be lowered to agronomically acceptable levels.

Agronomic evaluation of the obtained transformed plants and the resulting hybrids shows that these plants have good yields when compared to hybrids obtained from crossing plants not transformed with the s-gt antisense chimeric gene.

Additional plants wherein the invention is applicable are all other plants wherein a reduced expression of an UDP-glucose:thiohydroximate S-glucosyltransferase is desired, or wherein reduction of glucosinolate content is desired in view of the negative effects of the glucosinolates on animal or human consumption. Exemplary crops wherein the above embodiments are as well applicable are other oilseed rape species and other Crucifer crops, particularly other Brassica crops, including but not limited to: B. oleraceae, B . campestris, B. rapa, B. juncea, and B. nigra. Because of the large sequence similarity between the different S-GT isoforms and their encoding genes, it is believed that the constructs of Example 3 can equally well be used to obtain a reduced glucosinolate content in these other Brassica plants, preferably in their seeds.

The examples and embodiments of this invention described herein are only supplied for illustrative purposes. Many variations and modifications in accordance with the present invention are known to the person skilled in the art and are included in this invention and the scope of the claims. For instance, it is possible to alter, delete or add some nucleotides or amino acids to the DNA and protein sequences of the invention without departing from the essence of the invention.

All publications (including patent publications) referred to in this application are hereby incorporated by reference.

## Claims

1. A plant, transformed to contain a chimeric gene comprising:
(a) a plant-expressible promoter,
(b) a transcribed region operably linked to said promoter, comprising a DNA sequence encoding an RNA or protein, wherein said RNA or protein interfere with the normal expression of the UDP-glucose:thiohydroximate S-glucosyltransferase gene (s-gt gene) in cells of said plant, and
(c) a 3' transcription termination and polyadenylation region active in said plant.

2. The plant of claim 1, wherein said transcribed region comprises a DNA sequence selected from the following:
1) a DNA encoding an antisense RNA which is at least 80 % complementary, preferably at least 90 % complementary to the sense RNA encoded by an s-gt gene in a plant, or which has at least 80 %, preferably at least 90 %, sequence similarity to parts of said sense RNA of at least 100 nucleotides, preferably of at least 500 nucleotides;
2) a DNA encoding an antisense RNA which is at least 80 % complementary, preferably at least 90 % complementary, to at least 100 nucleotides, preferably at least 500 nucleotides, of the RNA encoded by the s-gt gene, the cDNA of which is comprised in plasmid pGL9, deposited in E. coli WK6 at the BCCM-LMBP under deposit number 3344;
3) a DNA encoding an antisense RNA which is at least 80 % complementary, preferably at least 90 % complementary, to at least 100 nucleotides, preferably at least 500 nucleotides, of the RNA encoded by the s-gt gene, the cDNA of which has the sequence of SEQ ID No. 28, or a DNA having substantial sequence similarity thereto.

3. The plant of claim 1, wherein said transcribed region comprises a DNA sequence encoding a sense RNA which has at least 80 % sequence similarity, preferably at least 90 % sequence similarity, more preferably at least 95 % sequence similarity, to a region of at least 100 nucleotides, preferably a region of at least 500 nucleotides, of the following RNAs:
1) an RNA encoded by the s-gt gene, the cDNA of which is comprised in plasmid pGL9, deposited in E. coli WK6 at the BCCM-LMBP under deposit number 3344, or
2) an mRNA, the cDNA of which is shown in SEQ ID No. 28.

4. The plant of claim 1, wherein said transcribed region comprises a DNA sequence encoding a ribozyme with a targetting region which is at least 90%, preferably at least 95 %, complementary to part of the following RNAs:
1) an RNA encoded by the s-gt gene characterized by the cDNA comprised in plasmid pGL9, deposited in E. coli WK6 at the BCCM-LMBP under deposit number 3344, or
2) an mRNA, the cDNA of which is shown in SEQ ID No. 28.

5. The plant of any one of claims 1 to 4, wherein said plant-expressible promoter is chosen from amongst the following group: a constitutive plant-expressible promoter, a 35S promoter, the promoter of the s-gt gene encoding an mRNA, the cDNA of which is comprised in E. coli WK6 deposited at the BCCM-LMBP under deposit number 3344, the promoter of the gene encoding an RNA, the cDNA of which has the sequence of SEQ ID No. 28, a pod tissue-specific promoter, a pod wall-specific promoter.

6. A DNA comprising a region encoding a protein with UDP-glucose:thiohydroximate S-glucosyltransferase activity, selected from the following:
1) a DNA encoding an mRNA, the cDNA of which is contained in plasmid pGL9, deposited in E. coli WK6 at the BCCM-LMBP under accession number 3344;
2) a DNA encoding an mRNA, the cDNA of which has the sequence of SEQ ID No. 28;
3) a DNA having substantial sequence homology to the DNA of SEQ ID No:28.

7. A DNA sequence encoding an antisense RNA selected from the following:
1) an antisense RNA which is complementary, preferably at least 90 % complementary, more preferably at least 95 % complementary, to a region of at least 100 nucleotides, preferably a region of at least 500 nucleotides, of an mRNA, the cDNA of which is contained in plasmid pGL9, deposited in E. coli WK6 at the BCCM-LMBP under accession number 3344;
2) an antisense RNA, which is at least 90 % complementary, more preferably at least 95 % complementary, to a region of at least 100 nucleotides, preferably a region of at least 500 nucleotides, of an mRNA, the cDNA of which comprises the coding region of SEQ ID No. 28; or
3) an antisense RNA encoded by the s-gt inhibitory gene contained in plasmid pTKV8a included in E. coli, MC1061, deposited a the BCCM-LMBP under accession number LMBP 3343, or an RNA having substantial sequence homology thereto.

8. A purified plant protein, characterized by having UDP-glucose:thiohydroximate S-glucosyltransferase activity and comprising:
1) any of the peptide fragments of SEQ ID No. 1 to 7; or
2) a region having at least 90 % sequence similarity, preferably at least 95 % sequence similarity, to any of the peptide fragments of SEQ ID Nos 1, 2, 3, 4, or 7.

9. The protein encoded by the cDNA contained in plasmid pGL9, deposited in E. coli WK6 at the BCCM-LMBP under accession number 3344, particularly the protein with the sequence of SEQ ID No. 28.

10. A DNA sequence encoding the protein of claims 8 or 9.

11. A plant, particularly a Brassica plant, more particularly a Brassica napus plant, transformed with any of the chimeric genes of claims 1 to 4, so to contain a level of total glucosinolates, preferably alkenyl glucosinolates, which is lower than 30 µmoles per gram dry defatted seed.

12. A Brassica napus plant, transformed so as to contain less than 30 µmoles, preferably less than 15 µmoles, particularly less than 5 µmoles, of alkenyl glucosinolates per gram of dry defatted seed.

13. A process for obtaining a Brassica napus plant having a significantly reduced expression of an s-gt gene, comprising the following steps:
a) transforming a plant cell with any of the chimeric genes of claims 1 to 4;
b) regenerating a plant from said transformed cell.

14. A process for obtaining a Brassica napus plant having a significantly reduced content of glucosinolates in its seeds, comprising the following steps:
a) transforming a plant cell with any of the chimeric genes of claims 1 to 4;
b) regenerating a plant from said transformed cell.

15. A seed, obtained from the plant of any of claims 1-4, and claims 11 or 12, comprising the chimeric gene of any of claims 1 to 4.

16. A seed cake, obtained by crushing the seed of claim 15.

17. The chimeric gene of any of claims 1 to 4.

18. A hybrid Brassica plant containing less than 30 µmoles of alkenyl glucosinolates per gram of dry defatted seed matter, obtained from two parent plants, at least one of which has total glucosinolate levels of more than 30 µmoles, preferably more than 50 µmoles, per gram dry defatted seed.

19. A hybrid Brassica plant containing less than 5 µmoles per gram of total glucosinolate levels in the whole seed basis, obtained from two parent plants, at least one of which has total glucosinolate levels of more than 5 µmoles, preferably more than 50 µmoles, per gram in the defatted seed meal.

20. A hybrid Brassica plant containing no detectable glucosinolates in the seeds, obtained from two parent plants, at least one of which has glucosinolate levels above 0 µmoles, preferably above 50 µmoles in the defatted seed meal.
